# EUROPÄISCHE PATENTSCHRIFT

(11) **EP 1 649 036 B1**
(45) Veröffentlichungstag und Bekanntmachung des Hinweises auf die Patenterteilung: **07.05.2008**
(21) Anmeldenummer: 04762549.6
(22) Anmeldetag: 30.07.2004
(51) Int. Cl.: C12P 21/00, C07K 1/107, C07K 5/12, C07K 7/06, C07K 7/50, C07K 7/64, A61K 38/12

(54) **VERFAHREN ZUR HERSTELLUNG ZYKLISCHER MOLEKÜLE**
METHOD FOR PRODUCING CYCLIC MOLECULES
PROCEDE POUR PRODUIRE DES MOLECULES CYCLIQUES

(30) Priorität: 31.07.2003 DE 10335584
(43) Veröffentlichungstag der Anmeldung: 26.04.2006
(73) Patentinhaber: ZYRUS Beteiligungsgesellschaft mbH & Co. Patente I KG, 12529 Schönefeld / OT Waltersdorf (DE)
(72) Erfinder: MARAHIEL, Mohamed, A., 35043 Marburg (DE); SIEBER, Stephan, La Scala Luxury Villas, San Diego, CA 92122 (US)
(74) Vertreter: Rohnke, Christian
(86) Internationale Anmeldenummer: PCT/DE2004/001704
(87) Internationale Veröffentlichungsnummer: WO 2005/012541

(56) Entgegenhaltungen:
- WO-A-01/05815
- WO-A-96/34878
- WO-A-98/28434
- WO-A-02/085401
- US-A1- 2002 192 773
- KOHLI RAHUL M ET AL: "Generality of peptide cyclization catalyzed by isolated thioesterase domains of nonribosomal peptide synthetases" BIOCHEMISTRY, Bd. 40, Nr. 24, 19. Juni 2001 (2001-06-19), Seiten 7099-7108, XP002304805 ISSN: 0006-2960
- TSENG CLAIRE C ET AL: "Characterization of the surfactin synthetase C-terminal thioesterase domain as a cyclic depsipeptide synthase." BIOCHEMISTRY, Bd. 41, Nr. 45, 12. November 2002 (2002-11-12), Seiten 13350-13359, XP002304806 ISSN: 0006-2960
- KEATING T A ET AL: "Chain termination steps in nonribosomal peptide synthetase assembly lines: directed acyl-S-enzyme breakdown in antibiotic and siderophore biosynthesis." CHEMBIOCHEM : A EUROPEAN JOURNAL OF CHEMICAL BIOLOGY. 2 FEB 2001, Bd. 2, Nr. 2, 2. Februar 2001 (2001-02-02), Seiten 99-107, XP002304807 ISSN: 1439-4227
- GRUENEWALD JAN ET AL: "Chemo- and regioselective peptide cyclization triggered by the N-terminal fatty acid chain length: The recombinant cyclase of the calcium-dependent antibiotic from Streptomyces coelicolor." BIOCHEMISTRY, Bd. 43, Nr. 10, 16. März 2004 (2004-03-16), Seiten 2915-2925, XP002304808 ISSN: 0006-2960

## Beschreibung

Auf der Suche nach neuen Medikamenten geraten Naturprodukte zunehmend in den Fokus der Wissenschaft und dienen ihr als Leitstruktur für die Entwicklung neuer Wirkstoffe. Diese pharmakologisch relevanten Moleküle werden von Bakterien oder Pilzen synthetisiert, und ihr Wirkungsspektrum reicht von
- antibiotischen (Infektionskrankheiten) über
- zytostatischen (Krebs) bis zu
- immunsuppressiven (Organtransplantation) Eigenschaften.
Die Synthese dieser kleinen Moleküle erfolgt in der Natur meist an großen Multienzymen, die hauptsächlich Peptide, Polyketide oder ein Hybrid aus beiden herstellen. Prominente Beispiele für solche Verbindungen sind Penicillin, Cephalosporin, Daptomycin, Epothilon und Cyclosporin, die zum Teil schon seit langer Zeit erfolgreich in der Medizin eingesetzt werden. Eine gemeinsame Eigenschaft dieser Verbindungen ist die zyklische Struktur, die für die biologische Aktivität entscheidend ist. Viele der oben genannten Verbindungen besitzen keine oder eine stark verminderte Wirksamkeit, wenn sie linear vorliegen. Im Gegensatz zu linearen Molekülen ist bei zyklischen Molekülen die konformatorische Flexibilität (die freie Bewegung und Rotation) durch den Ringschluss vermindert, was nur die biologisch aktive Form in Erscheinung treten lässt. Die Natur hat hier eine interessante Strategie gewählt, die sicherstellt, dass das synthetisierte Molekül in nur einer Modifikation vorkommt und daher spezifisch mit nur einem "Target" (Angriffsziel) im biologischen System interagiert. Angriffsziele sind meistens essentielle Bestandteile oder Funktionen einer Zelle, die für ihr Überleben wichtig sind, wie z.B. die Zellwand oder die Proteinsynthese. Da diese Moleküle selektiv bakterielle, fungale oder cancerogene (Krebs-) Zellen bzw. Viren eliminieren und gleichzeitig körpereigenes Zellgewebe verschonen, sind sie in der Therapie von Infektionskrankheiten und Krebs von enormer Bedeutung. Daneben können sie auch die von T-Zellen verursachte Immunabwehr unterdrücken, was die Organabstoßung bei Transplantationen wirksam verhindert (Cyclosporin).
Durch den intensiven Einsatz in der Medizin haben aber leider viele dieser Verbindungen ihre Wirksamkeit verloren, da die zu bekämpfenden Systeme Resistenzmechanismen entwickelt haben. Darüber hinaus besitzen viele potente Wirkstoffe sehr starke Nebenwirkungen, was ihren medizinischen Einsatz einschränkt (z.B. Nierentoxizität von Bacitracin). Daher besteht ein großer Bedarf an neuen bzw. optimierten Chemotherapeutika -(Antibiotika, Cytostatika und Immunsuppressiva), die möglichst wenige Nebenwirkungen aufweisen und hoch spezifisch mit ihrem "Target" interagieren. Für die Identifizierung solcher neuen Wirkstoffe können die bereits bekannten, potenten zyklischen Naturprodukte als Leitstruktur dienen und systematisch verändert und auf verbesserte Wirksamkeit getestet werden.
Derartige Naturstoffe werden im biologischen System von nicht ribosomalen Peptidsynthetasen (NRPS) hergestellt und von sogenannten Thioesterasen/Zyklasen zyklisiert, die sich in guter Ausbeute rekombinant in vitro überproduzieren lassen. Diese Enzyme können zuverlässig und effizient lineare Peptide einer bestimmten Leitstruktur in zyklische Moleküle überführen. Die Triebkraft der Zyklisierungsreaktion im natürlichen System ist die Aktivierung des C-Terminus (d. h. der freien Carbonsäure des linearen Peptids) durch eine Thioesterabgangsgruppe, dem Kofaktor Phosphopantethein. Im artifiziellen System wird die rekombinante Zyklase mit einem verkürzten Thioester-Mimic dieses natürlichen Kofaktors umgesetzt (N-Acetylcysteamin, SNAC). Unter verkürzten Thioester-Mimics sind dabei Substanzen zu verstehen, die
- die Funktion des natürlichen Kofaktors imitieren, aber nicht natürlichen Ursprungs sind,
- eine Thio-Abgangsgruppe besitzen und
- deren aliphatische Kette kürzer ist als die des natürlichen Kofaktors Phosphopantethein.

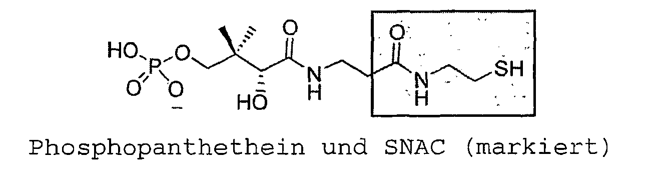

Mit der SNAC-Abgangsgruppe konnten bisher die Tyrocidin- und Surfactin- Zyklasen charakterisiert werden. Viele andere biologisch relevante zyklische Verbindungen, wie z.B. Fengycin, Mycosubtilin, Syringomycin und Bacitracin zeigen bei Verwendung der SNAC-Abgangsgruppe keine Zyklisierungsaktivität mit dem jeweiligen Enzym, was mit einer inkorrekten Faltung des Enzyms erklärt wird. Andere Verbindungen, wie z.B. CDA (Calcium dependent antibiotic) und Bacillibactin zeigen zum Teil sehr schlechten Umsatz mit den bekannten Substratanaloga.

Gegenstand der vorliegenden Erfindung sind nicht natürliche, synthetische Kofaktoren, deren chemische Abgangsgruppenqualitäten für eine effiziente Enzymacylierung sorgen. Entgegen der allgemein in der Fachwelt verbreiteten Annahme findet keine "Erkennung" des natürlichen Kofaktors Panthethein durch das Enzym statt, weshalb einzig das chemische Übertragungspotenzial des Acylrestes auf das aktive Zentrum im Enzym die entscheidende Größe ist. Die in der Fachwelt verbreitete Annahme, die "Erkennung" des natürlichen Kofaktors Panthethein durch das Enzym sei der Ausschlag gebende Faktor für die Zyklisierungsreaktion, ist beispielsweise dargestellt *in* JW Trauger, RM Kohli, HD Mootz, MA Marahiel and CT Walsh, Nature 2000, 407: 215-218*;* R Aggarwal, P Caffrey, PF Leadly, CJ Smith and J Staunton, Journal of the Chemical Society Communications 1995, 15: 1519-1520 sowie RS Gokhale, D Hunziker, DE Cane and C Khosla, Chemical Biology 1999, 6: 117-125*.*

Im Gegensatz zu den etablierten SNAC-Substraten weist z.B. Thiophenol als erfindungsgemäße ladungsstabilisierte Abgangsgruppe keinerlei strukturelle Ähnlichkeit mit dem natürlichen Kofaktor auf, besitzt aber eine deutlich bessere Abgangsgruppenqualität, da sich das Thiol in Konjugation mit einem aromatischen Benzolring befindet. Bei anderen erfindungsgemäßen Abgangsgruppen befindet sich die Thiol- oder Hydroxyfunktiön an einem sp³-Kohlenstoffatom, das direkt an den aromatischen Ring gebunden ist (α-C-Atom), so dass das aromatische System einen induktiven Effekt auf die Thio- oder Hydroxygruppe ausübt. Derartige erfindungsgemäße Abgangsgruppen werden nachfolgend als araliphatische Thio- oder Hydroxy-Abgangsgruppen bezeichnet. Dem Fachmann ist bekannt, dass sich der induktive Effekt eines aromatisches System stabilisierend auf die an ein α-C-Atom gebundenen Gruppen auswirkt und damit ihre Abgangsgruppenqualität erhöht. Dies kann z.B. in Michael B. Smith & Jerry March: March's Advanced Organic Chemistry. Reactions, Mechanisms, and Structure. 5th Edition 2000, John Wiley & Sons Inc., New York / Chichester / Brisbane / Toronto / Singapore nachgeschlagen werden. Im Falle des SNACs ist weder eine Konjugation mit einem aromatischen oder heteroaromatischen System noch eine Stabilisierung durch den induktiven Effekt eines aromatischen Systems in α-Stellung zum Kohlenstoffatom, an das die Thiogruppe gebunden ist, vorhanden, weshalb viele Enzyme keine Aktivität mit diesen Substraten zeigen oder niedrige k_{cat}/K_{M} Werte aufweisen.

### [Beschreibung und Stand der Technik]

Viele wertvolle Pharmazeutika besitzen zyklische Strukturen, wobei die Ringe dieser zyklischen Strukturen aus 5 oder mehr Atomen gebildet sind. Die aus dem Stand der Technik bekannten Methoden der synthetischen Chemie zur Darstellung zyklischer Verbindungen weisen zahlreiche Nachteile auf. Zu diesen Nachteilen gehören beispielsweise, aber nicht ausschließlich, geringe Ausbeuten der zyklischen Produkte, die Notwendigkeit von Schutzgruppen, um reaktive funktionelle Gruppen zu blockieren oder zu schützen, sowie die Notwendigkeit, diese Reaktionen in organischen Lösungsmitteln durchzuführen. Diese synthetischen Probleme können durch enzymatische Verfahren überwunden werden.
Die EP 0 832 096 B1 beschreibt ein Verfahren, mit dem ein nicht oxidiertes N-terminales Cystein eines ersten Oligopeptids mit dem C-terminalen Thioester eines zweiten Oligopeptids umgesetzt wird. Die Reaktion wird durch ein Thiol katalysiert, wobei die Thiogruppe direkt an einen aromatischen oder heteroaromatischen Ring gebunden ist. Dabei bildet sich ein β-Aminothioester als Zwischenprodukt, der sich spontan intramolekular umlagert, wobei die Amidbindung des Ligationsproduktes der beiden Oligopeptide entsteht. Nachteilig bei diesem Verfahren ist, dass das erste Oligopeptid zwingend ein N-terminales Cystein besitzen muss und dass es nicht für Zyklisierungsreaktionen geeignet ist.
Dagegen beschreibt die US 6,307,018 B1 eine allgemeine Methode, um ein erstes C-terminales α-Thioesterpeptid mit einem zweiten N-terminalen Aminosäure-Peptidsegment zu verbinden, bei der das N-terminale Aminosäure-Peptidsegment kein N-terminales Cystein besitzen muss. Allerdings muss das zweite Oligopeptid eine sekundäre Aminogruppe aufweisen, die über das N-Atom dieser sekundären Aminogruppe an eine nicht oxidierte Sulfhydrylgruppe eines aromatischen Thiols gebunden ist. Bei dem aromatischen Thiol kann es sich um Thiophenol, Benzylmercaptan oder ein S-Alkylbenzylmercaptan handeln. Darüberhinaus ist bei der US 6,307,018 B1 nachteilig, dass entweder der C-Terminus des ersten oder der N-Terminus des zweiten Oligopeptids Glycin sein muss. Die Methode ist nicht für die Zyklisierung von Peptiden geeignet.
Die US 2002/0192773 A1 beschreibt ein Verfahren zur enzymatischen Herstellung makrozyklischer Moleküle, bei dem rekombinante Thioesterase-Domänen (TE-Domänen, Zyklasen) aus einem PKS- oder NRPS-Multidomänensystem mit einem Substrat umgesetzt werden, wobei das Substrat einen über eine Thioesterabgangsgruppe aktivierten Acylrest und ein anhängendes Nucleophil enthält. Aktivierter Acylrest und Nucleophil sind über ein lineares Rückgrat voneinander getrennt. Nachteilig hierbei ist, dass die Abgangsgruppe nicht ladungsstabilisiert ist.

Durch eine unzureichende Zyklisierungsaktivität vieler Enzyme bei Verwendung strukturell analoger Abgangsgruppen zum natürlichen Kofaktor wie beispielsweise Coenzym A, Phosphopantethein und N-Acylcysteamin sind TE-Domänen in ihrer Anwendung jedoch stark limitiert. Die vorliegende Erfindung überwindet durch den Einsatz neuartiger Abgangsgruppen diese Einschränkung und ermöglicht nun die Erstellung von diversen zyklischen Wirkstoffbibliotheken vieler pharmakologisch bedeutender Molekülklassen.
Überraschend und im Widerspruch zum Stand der Technik wurde gefunden, dass die Erkennung der Substrate durch Enzyme bei der Zyklisierung von Peptiden und Proteinen keine Rolle spielt und dass ladungsstabilisierte Thio- und Hydroxyverbindungen gute Abgangsgruppen für die Acylierungsreaktion von Peptidzyklasen darstellen. Unter ladungsstabilisierten Thio- und Hydroxyverbindungen werden dabei aromatische oder heteroaromatische Ringsysteme verstanden, bei denen eine Hydroxy- oder Thiogruppe an eines der Ringatome oder an ein an das Ringsystem gebundenes Kohlenstoffatom gebunden ist. Die vorliegende Erfindung stellt Substrate bereit, mit deren Hilfe die enzymatische Zyklisierung von solchen Peptiden und Proteinen möglich ist, die nach dem Stand der Technik nicht der Zyklisierung zugänglich wären. Des weiteren kann die Ausbeute von Proteinen und Peptiden, die mit Methoden des Standes der Technik zyklisiert werden können, mit Hilfe der erfindungsgemäßen Substrate gesteigert werden. Darüber hinaus liefert die vorliegende Erfindung ein Verfahren, um weitere an der Zyklisierung von Peptiden und Proteinen beteiligte Substrate chemisch zu verändern und damit der Zyklisierung leichter zugänglich zu machen.

Im Stand der Technik beschreibt die WO 01/05815A die Herstellung eines Pseudomycin Kernes durch enzymatische Deacylierung von zyklische Depsipeptiden mittels einer ECB Deacylase bzw. Polymyxin Acylase. Des weiteren beschreibt dieses Dokument die Herstellung von Syringotmycin und entsprechende pharmazeutische Produkte. Es beschreibt die Synthese eines Substrates (peptideS-NAC) und anschließende Umsetzung dieses Substrates mit Peptid-Cyclasen zu einem zyklischen Peptid. WO 02/085401 A beschreibt Retrocycline, antivirale und antimikrobielle zyklische Peptide, die Herstellung von pharmazeutischen Verbindungen und ihre Anwendungen. US 2002/192773 A1 beschreibt ein Verfahren zur enzymatischen Herstellung makrozyklischer Moleküle, bei dem Rekombinante Thioesterase-Domänen (TE-Domänen, Cyclasen) aus einem PKS- oder NRPS-Multidomänensystem mit einem Substrat umgesetzt werden, wobei das Substrat einen über eine Thiosterabgangs-gruppe (SNAC) aktivierten Acylrest und ein anhängiges Nucleophil enthält. Aktivierter Acylrest und Nucleophil sind über ein lineares Rückrat voneinander getrennt.

### [Aufgabe der Erfindung]

Aufgabe der vorliegenden Erfindung ist es, ein alternatives Verfahren zur Herstellung von zyklischen Peptiden und insbesondere zur Herstellung eines Substrates und anschließende Umsetzung dieses Substrates in ein zyklisches Peptid mit Hilfe von Peptid-Cyclasen bereitzustellen und dabei zum Beispiel das Verfahren zur Darstellung zyklischer Peptide durch Umsetzung linearer Peptide mit Peptidzyklasen zu verbessern, wobei unter Verbesserung eine Erhöhung der Ausbeute an zyklischem Peptid und/oder eine Beschleunigung der Zyklisierungsreaktion und/oder eine Zyklisierung von mit bisherigen Verfahren nicht zyklisierbaren Peptiden verstanden wird. Diese Aufgabe wird erfindungsgemäß gelöst durch ein Verfahren zur Herstellung zyklischer Peptide, bei dem eine Peptidzyklase mit einem linearen Peptid in Kontakt gebracht wird, das lineare Peptid einen Acylrest enthält, der duch eine chemisch mit diesem Acylrest verbundene nucleophile Abgangsgruppe aktiviert ist, der aktivierte Acylrest des linearen Peptids das Zentrum der Peptidzyklase selektiv acyliert, wobei die nucleophile Abgangsgruppe unter Bildung des zyklischen Peptids abgespalten wird und zyklische Peptide mit Ringen aus mindestens 5 Atomen gebildet werden, wobei die nucleophile Abgangsgruppe, die mit dem Acylrest des linearen Peptids chemisch verbunden ist und diesen aktiviert, ladungsstabilisiert ist und die ladungsstabilisierte Abgangsgruppe an die Acylgruppe der C-terminalen Carbonsäure des Peptids gebunden ist. Unter "Substraten" werden hierbei lineare Peptide verstanden, an die eine nucleophile, ladungsstabilisierte erfindungsgemäße Abgangsgruppe chemisch gebunden ist. Unter ladungsstabilisierten Thio- und Hydroxyverbindungen werden dabei aromatische oder heteroaromatische Ringsysteme verstanden, bei denen eine Hydroxy- oder Thiogruppe an eines der Ringatome gebunden ist oder an ein Kohlenstoffatom, welches mit dem Ringsystem verbunden ist, wobei die chemische Struktur des aromatischen oder heteroaromatischen Systems so gewählt ist, dass eine an der Thio- oder Hydroxygruppe auftretende negative Ladung stabilisiert wird. Das erfindungsgemäße Verfahren führt zu höheren Ausbeuten an zyklischen Peptiden und / oder verbessert ihre Ausbeute und ermöglicht es erstmalig, auch Peptide wie Fengycin, Mycosubtilin, Syringomycin und Bacitracin zu zyklisieren, die mit den Methoden des Standes der Technik nicht zyklisierbar sind.

Die Bereitstellung der erfindungsgemäßen Substrate erfolgt durch die Synthese des linearen Peptids mit Hilfe von dem Fachmann bekannten Standardmethoden der Festphasenpeptidsynthese, darauf folgende Kupplung der freien Carbonsäure des linearen Peptids (freie Peptidsäure) an die erfindungsgemäße Thiol- oder Hydroxy-Abgangsgruppe, optionale Reinigung des so erhaltenen erfindungsgemäßen Substrates, darauf folgende Umsetzung des so erhaltenen erfindungsgemäßen Substrates mit einer Peptid-Zyklase und Reinigung des auf diese Weise erhaltenen zyklischen Peptids.

Dazu werden 1 Äquivalent (eq) der freien Peptidsäure mit 2 eq Dicyclohexylcarbodiimid (DCC), 2 eq N-Hydroxybenzotriazol (HOBt) und 10 eq der jeweiligen Abgangsgruppe versetzt und in THF für 30 min gerührt. Nach Zugabe von 0,5 eq Kaliumkarbonat wird die Reaktion für weitere 2,5 h agitiert und darauf filtriert, um ausgefallenen Dicyclohexylharnstoff (DCH) abzutrennen. Das Lösungsmittel wird am Rotationsverdampfer entfernt und das Peptid mit 95 % Trifluoressigsäure (TFA), 2,5 % Wasser und 2,5 % Triisopropylsilan für 3 h entschützt. Das Reaktionsgemisch wird darauf in eiskalten Diethylether gegeben, worauf das Substrat präzipitiert. Dieser Schritt stellt eine Reinigung dar, bei der Reaktionsnebenprodukte abgetrennt werden, und führt zu einer Reinheit des Substrates von bis zu 80%, was im Allgemeinen für die weitere Umsetzung des Substrates mit einer Peptidzyklase ausreichend ist. Optional kann die Reinheit des Substrat anschließend mittels präparativer HPLC erhöht werden.
Enthält das lineare Peptid neben der C-terminalen freien -COOH-Gruppe weitere freie COÖH-Gruppen innerhalb der Peptidkette, wie beispielsweise COOH-Gruppen von Glutaminsäure und / oder Asparaginsäure, so müssen diese nicht C-terminalen freien COOH-Gruppen vor der Umsetzung des linearen Peptids mit einem Aktivierungsreagenz mit einer geeigneten orthogonalen Schutzgruppe geschützt werden, die nach Darstellung des erfindungsgemäßen Substrates wieder abgespalten werden muss. Geeignete Schutzgruppen und geeignete Methoden zu deren Entfernung sind dem Fachmann bekannt und können beispielsweise in Theodora W. Greene and Peter G. M. Wuts, "Protective groups in organic synthesis", 2nd Edition 1991, John Wiley & Sons Inc., New York / Chichester / Brisbane / Toronto / Singapore nachgeschlagen werden.
Das gereinigte Substrat mit der erfindungsgemäßen Abgangsgruppe wird darauf mit der jeweiligen Peptidzyklase im Verhältnis 1 (Enzym): 100 (Substrat) in 25 mM HEPES, 50 mM NaCl bei pH 7 und Raumtemperatur für 30-60 min inkubiert. Die Herstellung der HEPES-Lösung ist dem Fachmann bekannt und wurde in J. Sambrook, E.F. Fritsch and T. Maniatis: Molecular Cloning: A Laboratory Manual Vol. I-III, Cold Spring Harbor Laboratory Press, 1982*,* beschrieben. Die Identifizierung und Quantifizierung des Reaktionsproduktes erfolgt mittels analytischer HPLC.

Alternativ zum Aktivierungsreagenz DCC lassen sich die erfindungsgemäßen Substrate auch durch Umsetzung der Peptidsäure mit der jeweiligen Abgangsgruppe in Gegenwart anderer, den C-Terminus der Peptidsäure aktivierenden Reagenzien umsetzen. Entsprechungen sind bekannt und können, ohne den Schutzbereich der Patentansprüche zu verlassen, verwendet werden. Hierzu zählen beispielsweise die dem Fachmann bekannten Aktivierungsreagenzien DCI, PyClop, HBTU, HATU, HOSu, TBTU, T3P, BopCl und 3-Cl-1-Pyridiniumiodid. Als Kupplungsadditive sind neben dem oben aufgeführten HOBt beispielsweise auch die dem Fachmann bekannten Substanzen HOAt und HONB verwendbar. Dem Fachmann ist bekannt, dass diese Reaktionen zweckmäßig unter Zugabe einer Base wie beispielsweise DIPEA durchgeführt werden. Dem Fachmann sind weiterhin verschiedene Lösungsmittel zur Verwendung in den genannten Verfahren bekannt. Er kann diese Kombinationen von Aktivierungsreagenzien, Kupplungsadditiven, Basen und Lösungsmitteln mit seinem üblichen Wissen und der Standardliteratur selbst herstellen.

Als ladungsstabilisierte Abgangsgruppen werden bei der vorliegenden Erfindung chemische Verbindungen verstanden, die eine Thio- oder Hydroxyl-Gruppe besitzen und bei denen das freie Elektronenpaar des durch die Acylierungsreaktion freigesetzten Thiolat- oder Hydroxylat-Ions in Konjugation mit weiteren Elektronenpaaren von beispielsweise, aber nicht ausschließlich, C=C- oder C=N-Doppelbindungen steht oder bei der die Thio- oder Hydroxygruppe an ein Kohlenstoffatom gebunden ist, das seinerseits an einen aromatischen oder heteroaromatischen Ring gebunden ist. Solche Verbindungen sind z.B. Oxo- und Thio-aromatische und -heteroaromatische Verbindungen, aber auch ladungsstabilisierte aliphatische Oxo- und Thio- Abgangsgruppen. Diese Abgangsgruppen, wie z.B. Thiophenol, Phenol, 2,3,4,5,6-Pentafluorphenol, die Mercaptoanisole und Thiokresole sowie 2-Hydroxypyridin und 2-Thio pyridin funktionieren für die Acylierungsreaktion von Peptidzyklasen, die keinerlei Ähnlichkeit mit dem natürlichen Kofaktor besitzen, und weisen verbesserte Eigenschaften für *in vitro* Zyklisierungsreaktionen auf.

Dies soll im Folgenden exemplarisch, aber nicht erschöpfend am Beispiel des Thiophenols erläutert werden:
Die Thiophenol-Abgangsgruppe weist bis auf die Thiolfunktion keine strukturelle Ähnlichkeit zum natürlichen 4'-Phosphopanthethein-Kofaktor auf. Die Thiolfunktion ist direkt an einen aromatischen Phenylring gebunden. Dieses Strükturmerkmal verursacht die gegenüber den bisher beschriebenen Abgangsgruppen höhere Reaktivität dieser Verbindung. Beim nukleophilen Angriff des aktivierten Ser (= Serin) der katalytischen Triade im aktiven Zentrum des Enzyms wird diese Abgangsgruppe als Thiophenolat-Ion freigesetzt. Die resultierende negative Ladung am Schwefelatom kann dabei sehr gut durch den angrenzenden Phenylring delokalisiert werden.

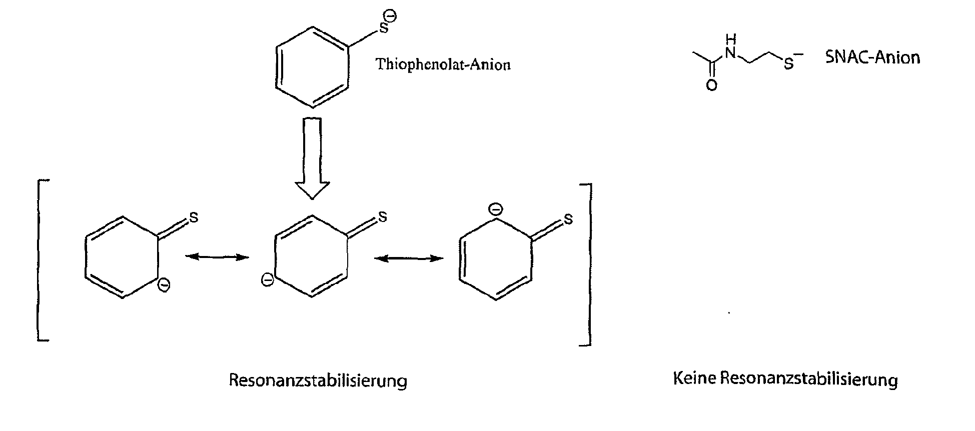

Eine derartige Erhöhung und Stabilisierung der Elektronendichte tritt bei den SNAC-, CoA- und Ppant-Abgangsgruppen nicht auf. Dort bleibt die negative Ladung am Schwefelatom lokalisiert. Da aber in der Regel die Güte einer Abgangsgruppe proportional zu ihrer chemischen Stabilisierung ist, sind SNAC-, CoA- und Ppant chemisch betrachtet schlechtere Abgangsgruppen als Thiophenol.
Dem Fachmann ist bekannt, dass das Austrittsvermögen und damit die Qualität einer Abgangsgruppe von der Fähigkeit der Abgangsgruppe abhängt, eine negative Ladung zu stabilisieren. Unter Stabilisierung einer Ladung versteht der Fachmann dabei das Verteilen von Ladungen oder Teilladungen auf mehrere Atome oder Bindungen, so dass diese Ladung oder Teilladung nicht an einem einzigen Atom oder an einer einzigen Bindung innerhalb eines Moleküls lokalisiert ist. Dabei sind dem Fachmann zwei verschiedene Möglichkeiten der Ladungsstabilisierung organischer Moleküle bekannt, die allgemein als mesomere oder Resonanz-Effekte (M-Effekte) und induktive Effekte (I-Effekte) bezeichnet werden. Unter einem mesomeren oder Resonanz-Effekt versteht der Fachmann das schnelle und reversible Verschieben von π-Elektronenpaaren, welches in Systemen auftritt, die konjugierte π-Bindungen besitzen. Dem Fachmann ist bekannt, dass der mesomere Effekt über große Entfernungen und damit viele Bindungen hinweg wirksam ist, wenn ein entsprechend ausgedehntes konjugiertes π-System vorliegt. In Ringverbindungen mit konjugierten π-Systemen nehmen auch Substituenten an der Mesomerie teil, sofern sie über freie π-Elektronenpaare verfügen oder diese aufnehmen können. Ist eine Ladung in einer substituierten Ringverbindung mit konjugiertem π-system und mesomeriefähigen Substituenten zu stabilisieren, so hängt es von der Position der Substituenten zueinander ab, ob und welche dieser Substituenten tatsächlich an der Ladungsstabilisierung durch Mesomerie teilnehmen. Dies ist dem Fachmann bekannt.
Besitzt ein Atom eine höhere Elektronegativität und damit eine stärkere Anziehung auf die Bindungselektronen als sein mit ihm über eine σ-Bindung verbundenes Nachbaratom, oder ist ein Atom mit weiteren Atomen oder Atomgruppen verbunden, die Elektronen ziehend wirken, so wird die Bindungselektronenwolke der hier betrachteten σ-Bindung in Richtung auf den Elektronenzug verschoben, d.h. polarisiert. Diese Polarisation einer σ-Bindung wird als Teilladung bezeichnet, da es sich hierbei um eine geringfügige Verschiebung von Elektronenwolken handelt und diese Verschiebung nicht zum Auftreten ganzzahliger Vielfacher der Elementarladung an einem bestimmten Atom führt. Die durch unterschiedliche Elektronegativitäten und / oder unterschiedlichen Elektronenzug von Atomen und Atomgruppen hervor gerufene Polarisation von σ-Bindungen wird vom Fachmann auch als induktiver Effekt bezeichnet. Dem Fachmann ist bekannt, dass der induktive Effekt für einander benachbarte Bindungen am größten ist und mit zunehmender Entfernung zu dem Atom oder der Atomgruppe, das / die ihn hervor ruft, rasch abnimmt. Dies kann z.B. in Michael B. Smith & Jerry March: March's Advanced Organic Chemistry. Reactions, Mechanisms, and Structure. 5th Edition 2000, John Wiley & Sons Inc., New York / Chichester / Brisbane / Toronto / Singapore nachgeschlagen werden.
Der Fachmann unterscheidet positive und negative mesomere bzw. induktive Effekte. Als positiv wird ein solcher Effekt bezeichnet, wenn er die Elektronendichte in Form einer Ladung oder Teilladung an einem Atom oder einer Atomgruppe erhöht (+M-Effekt, +I-Effekt), als negativ, wenn er die Elektronendichte verringert (-M-Effekt, -I-Effekt). Befinden sich beispielsweise an einem aromatischen System mehrere Substituenten, so üben sie ihre M- und I-Effekte unabhängig voneinander aus und können einander verstärkend, aber auch gegenläufig in Bezug auf die Ladungsstabilisierung an einem bestimmten Atom wirken. In der Regel wirken mesomere Effekte stärker als induktive.
In der vorliegenden Erfindung werden daher bevorzugt solche ladungsstabilisierten Abgangsgruppen gewählt, bei denen eine Hydroxy- oder Thiogruppe an eines der Ringatome eines aromatischen, heteroaromatischen oder araliphatischen Systems gebunden ist oder an ein Kohlenstoffatom, welches mit dem Ringsystem verbunden ist, wobei die chemische Struktur des aromatischen, heteroaromatischen oder araliphatischen Systems so gewählt ist, dass die Summe der mesomeren und induktiven Effekte der enthaltenen funktionellen Gruppen einen Elektronenzug auf das Thiolat- oder Hydroxylat-Ion ausübt und auf diese Weise dessen negative Ladung stabilisiert.

Ein weiteres wichtiges Kriterium zur Quantifizierung der Abgangsgruppenqualität ist der pK_{A}-Wert einer chemischen Verbindung: Je höher der pK_{A}-Wert, desto schlechter ist die jeweilige Abgangsgruppe. CoA, Ppant und SNAC haben pK_{A}-Werte von 10 - 11, während Thiophenol einen pK_{A}-Wert von 8 aufweist. Es lässt sich somit sagen, dass Thiophenol seine mangelnde strukturelle Überstimmung zum natürlichen Phosphopanthethein-Kofaktor überraschend und im Widerspruch zum Stand der Technik durch seine hohe chemische Reaktivität überkompensieren kann, was auch für andere aromatische, heteroaromatische und ladungsstabilisierte araliphatische Thiol- und Hydroxyl-Verbindungen gilt. Dabei werden vorteilhaft solche ladungsstabilisierten aromatische, heteroaromatische und araliphatischen Thiol- und Hydroxyl-Verbindungen als Abgangsgruppen verwendet, deren pK_{A}-Wert kleiner oder gleich 10, bevorzugt kleiner oder gleich 8 ist. Die Ringsysteme der erfindungsgemäßen aromatischen, heteroaromatischen und araliphatischen Thiol- und Hydroxyverbindungen können durch einen oder mehrere Substiuenten mit positiven oder negativen induktiven oder mesomeren Effekte substituiert sein, wobei die Gesamtheit der Effekte aller vorhandenen Substituenten elektronenziehend und damit ladungsstabilisierend auf das bei der enzymatischen Zyklisierung frei gesetzte Thiolat- oder Hydroxylation wirkt.

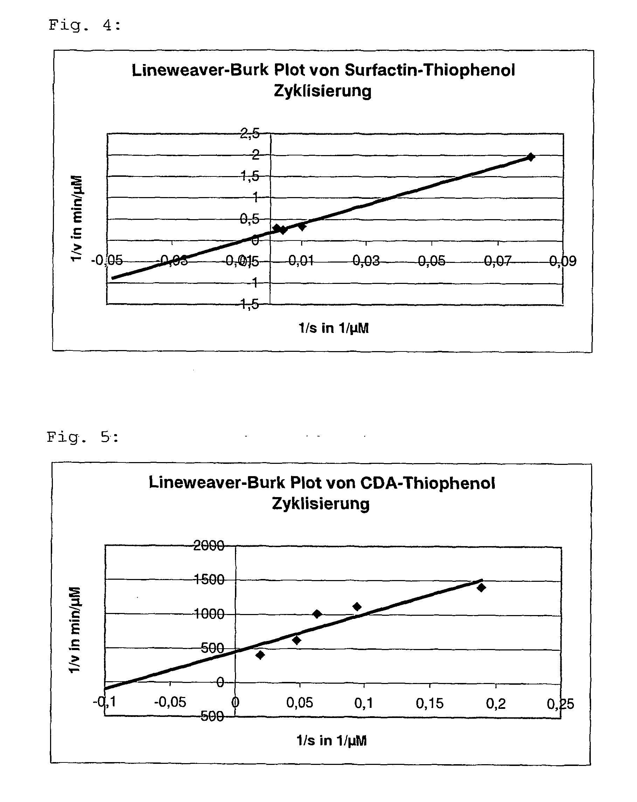

Bei Verwendung von ladungsstabilisierten Thiol- und Hydroxyverbindungen zeigen auch solche Enzyme Zyklisierungsaktivität, die bei Verwendung der bisher bekannten Abgangsgruppen als inaktiv klassifiziert wurden (ca. 2/3 aller bisher untersuchten). Enzyme, die auch bei Verwendung von SNAC als Abgangsgruppe zyklisieren, zeigen verbesserte kinetische Eigenschaften mit bis zu 15 fach gesteigerten k_{cat}/K_{M} Werten bei gleich bleibender Regio- und Stereoselektivität, wenn Thiophenol-Derivate an Stelle von SNÄC-Abgangsgruppen verwendet werden. Dies konnte am Beispiel des Surfactin-Thiophenols gezeigt werden (siehe Fig. 4). Surfactin zeigt ebenfalls verbesserte Reaktionsgeschwindigkeiten bei der Zyklisierung, wenn o-, m- oder p-Mercaptoanisol bwz. o-, m- oder p-Thiokresol als Abgangsgruppe verwendet werden.
Die Katalyse von Peptid-Zyklasen lässt sich in zwei Teilschritte zerlegen:
- Der erste Teilschritt ist die Ausbildung des Peptidyl-o-TE-Intermediates durch Acylierung des aktivierten Ser-Restes der katalytischen Triade.
- Der zweite Teilschritt ist die Deacylierung des Ser-Restes durch eine funktionelle Gruppe der gebundenen Peptidkette als internes Nukleophil.
Thioestergebundene Abgangsgruppen können ausschließlich die katalytische Effizienz des ersten Teilschrittes beeinflussen: die Bildung des Peptidyl-O-TE-Intermediates. Experimente mit der neuen Abgangsgruppe Thiophenol bestätigen dies (siehe Fig. 4 bis Fig. 6). Eine Mutation im aktiven Zentrum des Enzyms zeigt keine Aktivität, was die abgangsgruppenbedingte Acylierung und darauffolgende enzymatische Zyklisierung bestätigt.

Folgende aromatische, heteroaromatische und araliphatische Grundelemente dienen als ladungsstabilisierte Abgangsgruppen: 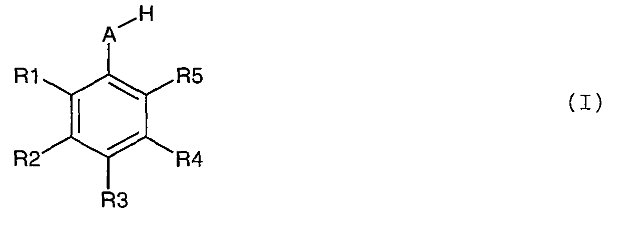 mit
A = O, S und
sowie R1, R2, R3, R4 und R5, die unabhängig voneinandersind: -NO₂, -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂⁻ -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind, wobei die Bedingungen so gewählt sind, dass bei Temperaturen unter 200 °C und Atmosphärendruck keine explosiven Stoffe entstehen und die Verbindung bestehend aus linearen Peptiden und daran gebundenen erfindungsgemäßen Abgangsgruppen bei diesen Bedingungen nicht hydrolytisch gespalten werden, 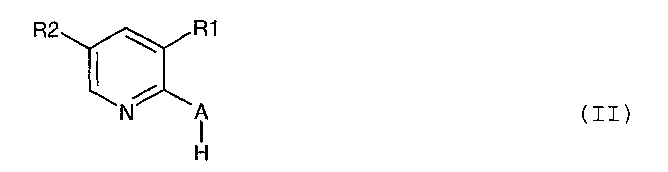 mit
A = O, S und
sowie R1 und R2, die unabhängig voneinander sind:
-NO₂, -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂⁻, -Alkyl, -Alkenyl, -Cycloalkyl, - Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind, wobei die Bedingungen so gewählt sind, dass bei Temperaturen unter 200°C und Atmosphärendruck keine explosiven Stoffe entstehen und die Verbindung bestehend aus linearen Peptiden und daran gebundenen erfindungsgemäßen Abgangsgruppen bei diesen Bedingungen nicht hydrolytisch gespalten werden, wobei dem Fachmann bekannt ist, dass Substituenten an C-4 oder C-6 des Pyridinringes keine Ladungsstabilisierung des an C-2 befindlichen Hydroxy- oder Thiol-Slibstitueritenbewirken, 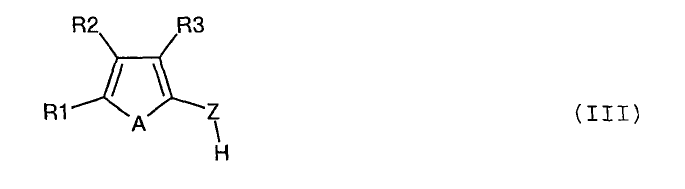 mit
A = O , S, und
Z = O, S,
sowie R1, R2, und R3, die unabhängig voneinander sind:
-NO₂, -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂⁻, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind, wobei die Bedingungen so gewählt sind, dass bei Temperaturen unter 200 °C und Atmosphärendruck keine explosiven Stoffe entstehen und die Verbindung bestehend aus linearen Peptiden und daran gebundenen erfindungsgemäßen Abgangsgruppen bei diesen Bedingungen nicht hydrolytisch gespalten werden, 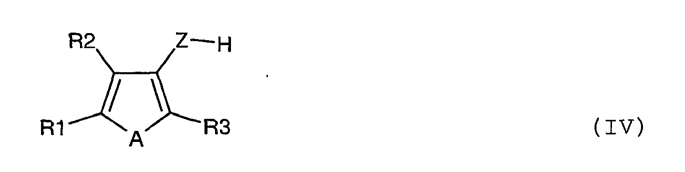 mit
A = O, S, und
Z = O, S,
sowie R1, R2, und R3, die unabhängig voneinander sind:
-NO₂ -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂⁻, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sein können, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind, wobei die Bedingungen so gewählt sind, dass bei Temperaturen unter 200 °C und Atmosphärendruck keine explosiven Stoffe entstehen und die Verbindung bestehend aus linearen Peptiden und daran gebundenen erfindungsgemäßen Abgangsgruppen bei diesen Bedingungen nicht hydrolytisch gespalten werden, 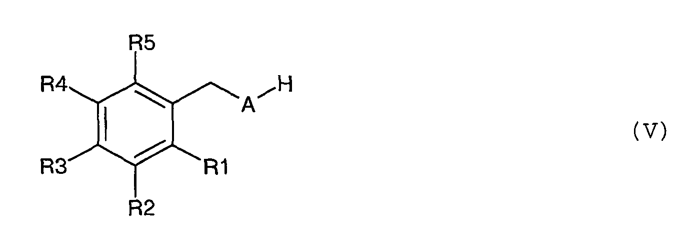 mit
A = O , S und
sowie R1, R2, R3, R4 und R5, die unabhängig voneinander sind:
-NO₂, -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂-, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind, wobei die Bedingungen so gewählt sind, dass bei Temperaturen unter 200 °C und Atmosphärendruck keine explosiven Stoffe entstehen und die Verbindung bestehend aus linearen Peptiden und daran gebundenen erfindungsgemäßen Abgangsgruppen bei diesen Bedingungen nicht hydrolytisch gespalten werden,

Des weiteren können diese Abgangsgruppen den natürlichen Kofaktor auch für andere artifizielle Reaktionen *in vitro* mit Enzymen der nichtribosomalen Peptidsynthetasen ersetzen. Eine solche Reaktion ist die Kondensationsreaktion zur Knüpfung einer Peptidbindung, katalysiert durch die Kondensationsdomäne (C-Domäne), die auch mit Thioester-gebundenen Substraten arbeitet.

Überraschend und im Widerspruch zum Stand der Technik wurde gefunden, dass die Erkennung eines Substrates durch das jeweilige Enzym keine Rolle spielt. Die vorliegende Erfindung stellt somit eine neue und für den Durchschnittsfachmann überraschende Weiterentwicklung des in der US 2002/0192773 A1 beschriebenen Verfahrens zur enzymatischen Herstellung makrozyklischer Moleküle dar, bei dem gereinigte isolierte Thioesterase-Domänen (TE-Domänen, Zyklasen) aus einem PKS- oder NRPS-Multidomänensystem mit einem Substrat umgesetzt werden. Bei den in Frage kommenden Substraten handelt es sich um lineare Peptide und Lipopeptide mit 5 - 22 monomeren Baueinheiten wie z.B. Aminosäuren. Substrate sind beispielsweise Fengycin, Mycosubtilin, Bacillibactin, CDA, Surfactin, Bacitracin oder Syringomycin und weitere Substrate, die bereits in US 2002/0192773 A1 beschrieben wurden, sowie Pristinamycin, wobei die genannten Substrate zusätzlich eine erfindungsgemäße Abgangsgruppe aufweisen. Einige dieser Substrate sind nachfolgend dargestellt:

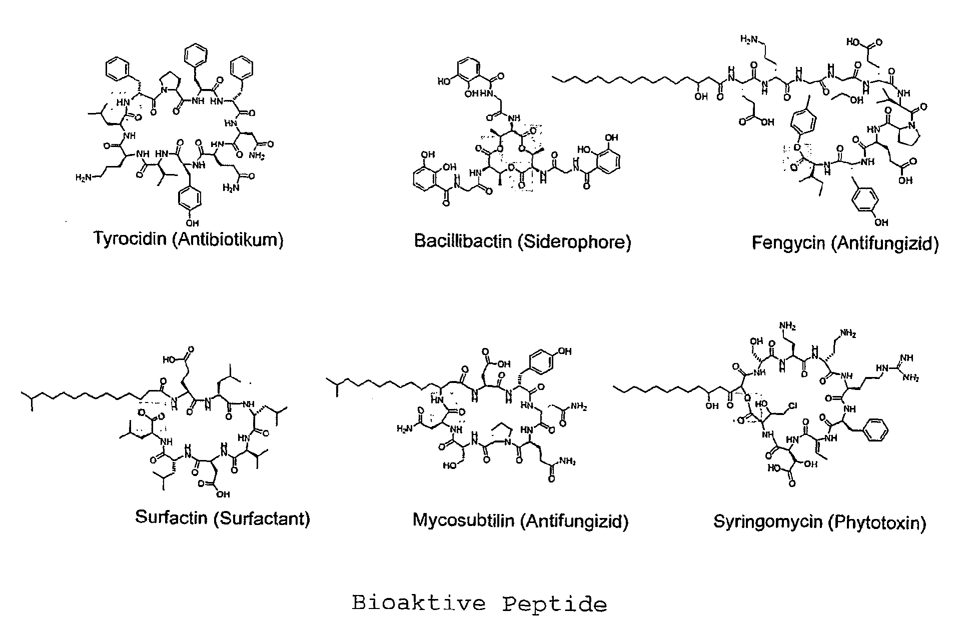

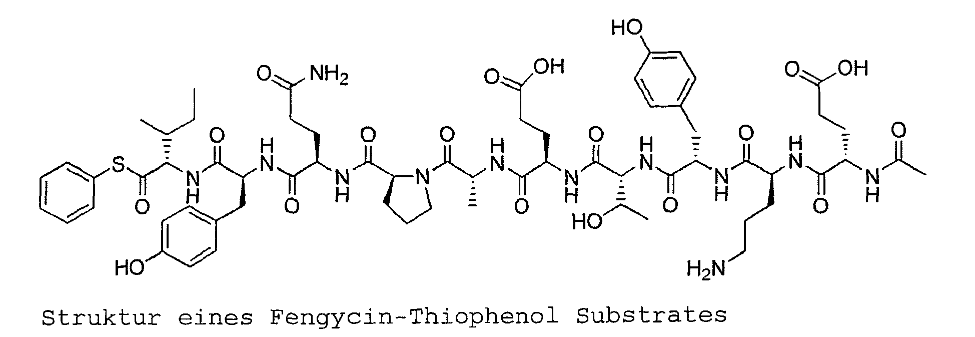

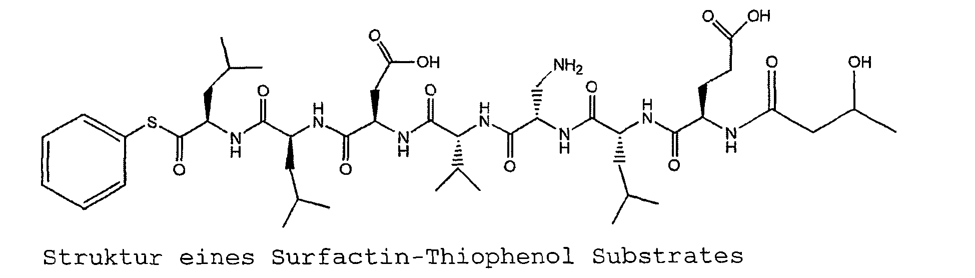

Das erfindungsgemäße Verfahren stellt im Vergleich zum Stand der Technik auch bei solchen linearen Peptiden eine Verbesserung dar, die bereits mit dem Fachmann bekannten Methoden zyklisiert werden konnten, da das erfindungsgemäße Verfahren die Reaktionsgeschwindigkeit der Zyklisierung beschleunigt und / oder zu höheren Ausbeuten des zyklischen Peptids führt.

Bei den in Frage kommenden Enzymen handelt es sich um gereinigte isolierte Thioesterase-Domänen oder Peptid-Zyklasen aus NRPS- oder PKS-Systemen, wie beispielsweise den entsprechenden Domänen bzw. Zyklasen von Fengycin, Mycosubtilin, Bacillibactin, CDA, Surfactin, Bacitracin, Syringomycin, Tyrocidin, Pristinamycin und allen übrigen in US 2002/0192773 A1 aufgeführten Peptid-Zyklasen, Thioesterasen und gereinigten isolierten Thioesterasen.

Das lineare Peptid enthält proteinogene und nichtproteinogene Aminosäuren in seinem Rückgrat. Eingebettet in dieses Rückgrat können auch Reste und / oder funktionelle Gruppen sein, die sich nicht von Aminosäuren ableiten wie z.B. gesättigte oder ungesättigte Kohlenstoff-Spacer. Die fakultativ in das Rückgrat eingebetteten Reste und /oder funktionellen Gruppen wurden bereits in US 2002/0192773 A1 beschrieben. Die erfindungsgemäße Abgangsgruppe wird dabei entweder an der C-terminalen Carbonsäuregruppe oder einer Seitenketten-Carbonsäure angebracht.

Die erfindungsgemäße Abgangsgruppentechnologie kann zur Herstellung von Stoffbibliotheken für zyklische Peptide und Proteine verwendet werden, indem neue Substratvarianten strukturell bedeutsamer Moleküle (beispielsweise Fengycin, Mycosubtilin, Syringomycin, CDA, etc.), die bisher keine oder nur geringe Aktivität mit der herkömmlichen Abgangsgruppe SNAC zeigten, hergestellt und auf verbesserte biologische Eigenschaften (antibiotisch, antiviral, antifungal, cytostatisch) getestet werden. Die Substratvarianten werden durch kombinatorische Festphasenpeptidsynthese hergestellt und nach der oben aufgeführten allgemeinen Vorschrift mit den neuen Abgangsgruppen versehen. Bevorzugt wird dabei eine Stoffbibliothek für auf Zielzellen angepasste Peptidantibiotika hergestellt, wobei es sich um zyklische Peptidantibiotika handelt, die mit Hilfe des erfindungsgemäßen Verfahrens hergestellt wurden.

Das erfindungsmäße Verfahren kann zur Herstellung von Zyklisierungs-Kits verwendet werden, die Mittel zur Kopplung erfindungsgemäßer ladungsstabilisierter Abgangsgruppen sowie Peptidzyklasen bereit stellen, so dass lineare Peptide mit den bereit gestellten Abgangsgruppen zunächst zu erfindungsgemäßen Substraten und anschließend mit den bereit gestellten Peptidzyklasen zu zyklischen Peptiden umgesetzt werden können. Dem Hersteller des erfindungsgemäßen Kits ist aus seinem allgemeinen Wissen bekannt, wie er die einzelnen Komponenten des Kits, z.B. die Puffer, herstellt, formuliert und lagert.

Die mit dem erfindungsgemäßen Verfahren hergestellten zyklischen Peptide und Proteine können als Arzneimittel für Patienten zur Therapie, Diagnostik und Prophylaxe von Erkrankungen verwendet werden, bei denen bakterielle und / oder virale Infektionen auftreten. Die erfindungsgemäßen zyklischen Peptide und Proteine können, sofern sie cytostatische und / oder immunsuppressive Eigenschaften besitzen, ferner als Arzneimittel für Patienten zur Therapie, Diagnostik und Prophylaxe von Tumorerkrankungen sowie in der Transplantationsmedizin verwendet werden. Der Begriff Patient bezieht sich dabei gleichermaßen auf Menschen und Wirbeltiere. Damit können die Arzneimittel in der Human- und Veterinärmedizin verwendet werden. Pharmazeutisch akzeptable Kompositionen von Verbindungen gemäß den Ansprüchen können als Di- bis Oligomere oder als deren Salze, Ester, Amide oder "Prodrugs" vorliegen, sofern sie nach zuverlässiger medizinischer Beurteilung keine übermäßige Toxizität, Irritationen oder allergische Reaktionen am Patienten auslösen. Die therapeutisch wirksamen Verbindungen der vorliegenden Erfindung können dem Patienten als Teil einer pharmazeutisch akzeptablen Komposition entweder oral, rektal, parenteral, intravenös, intramuskulär, subkutan, intracisternal, intravaginal, intraperitoneal, intravasculär, intrathekal, intravesikal, topisch, lokal (Puder, Salbe oder Tropfen) oder in Sprayform (Aerosol) verabreicht werden. Die intravenöse, subkutane, intraperitoneale oder intrathekale Gabe kann dabei kontinuierlich mittels einer Pumpe oder Dosiereinheit erfolgen. Dosierungsformen für die örtliche Administration der erfindungsgemäßen Verbindungen schließen Salben, Puder, Zäpfchen, Sprays und Inhalationsmittel ein. Die aktive Komponente wird dabei unter sterilen Bedingungen mit einem physiologisch akzeptablen Trägerstoff und möglichen Preservativen, Puffern, Verdünnungs- und Treibmitteln je nach Bedarf vermischt.

### [Beispiele]

### Ausführungsbeispiel 1: Herstellung und Reinigung des Fengycin-Thiophenol-Substrates sowie Zyklisierung

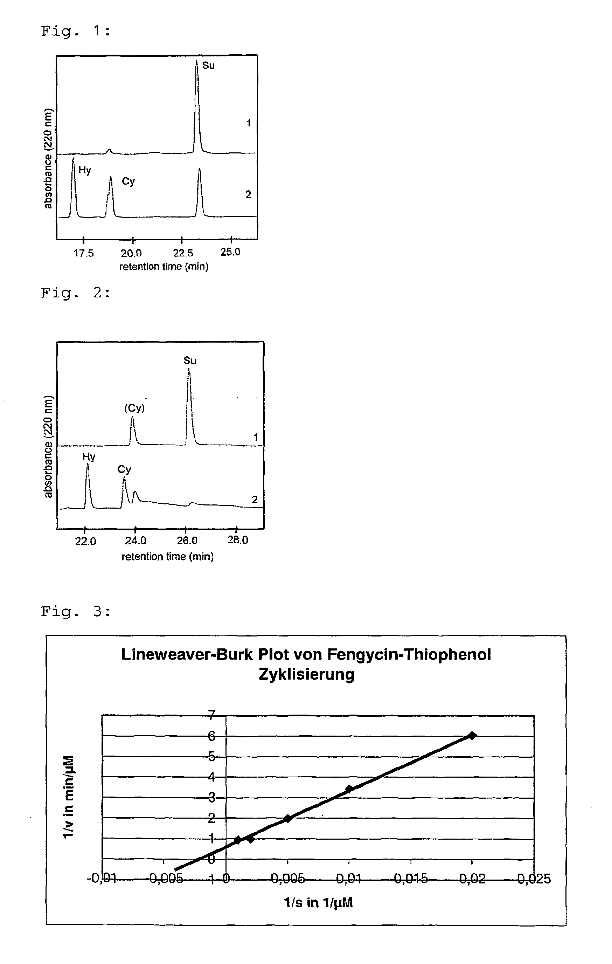

Das lineare Fengycin-Substrat wird zunächst nach Standardmethoden der Peptidfestphasensynthese hergestellt. Die Peptidsequenz lautet: Acetyl-Glu-D-Orn-Tyr-D-Thr-Glu-D-Ala-Pro-Gln-D-Tyr-Ile-COOH. Im nächsten Schritt werden 0,05 mmol des Peptides mit 0,1 mMol DCC, 0,1 mMol HOBt und 0,5 mMol Thiophenol versetzt und in 2 ml THF gelöst. Das Gemisch rührt für 30 min bei RT, und 0,05 mMol Kaliumkarbonat werden addiert. Das Gemisch rührt für weitere 2,5 h bei RT, und darauf wird durch Filtration festes DCH abgetrennt und das Lösungsmittel am Rotationsverdampfer entfernt. Die Entschützung der Peptidseitenketten erfolgt für 3 h in 2 ml 95% TFA, 2,5% Wasser und 2,5% Triisopropylsilan. Das Gemisch wird dann in 50 ml eiskalten Diethylether geschüttet und der entstehende Feststoff durch Zentrifugation abgetrennt. Die Reinigung des Feststoffs erfolgt mittels präparativer HPLC mit einer C₁₈-Nucleodursäule (Porengröße 100 Å, Partikelgröße 7 µM, Durchmesser 10 mm, Länge 250 mm, Macherey-Nagel) mit einem Gradienten von 10% Acetonitril in Wasser/0,1 % TFA auf 70 % Acetonitril in Wasser/0,1% TFA in 40 min bei einer Flussrate von 6 ml/min. Die Retentionszeit des zyklisierten Fengycins (vgl. Fig. 1) beträgt 19 min. Die Ausbeute beträgt zwischen 70 und 80 %.
Die Produkte werden mit LC-MS und MALDI-TOF Massenspektrometrie auf Reinheit und Identität überprüft.
Die Zyklisierung des linearen Fengycin-Thiophenol-Substrates erfolgt in einem wässrigen Zyklisierungspuffer bestehend aus 2-[4-(2-Hydroxyethyl)-1-piperazinyl]-ethansulfonsäure (HEPES, 25 mM) und Natriumchlorid (NaCl, 50 mM) bei pH 7 in einem Gesamtvolumen von 50 µL. Die Substratkonzentration beträgt 100 µM für Standardzyklisierungsreaktionen. Die Zyklisierungsreaktion wird durch Zugabe von rekombinanter Fengycin TE bei einer Endkonzentration von 5 µM initiiert und durch Zugabe von 35 µL 4%-iger Trifluoressigsäure (TFA) in Wasser nach 4 Stunden gestoppt. Im Anschluss werden die Reaktionsprodukte mittels HPLC mit einer C₁₈-Nucleodursäule (Porengröße 100 Å, Partikelgröße 3 µM, Durchmesser 10 mm, Länge 250 mm, Macherey-Nagel) und einem Gradienten von 30 % Acetonitril in Wasser / 0,1 % TFA auf 60 % Acetonitril in Wasser / 0,1 % TFA in 35 min bei einer Flussrate von 0,4 mL/min bei 40 °C untersucht. Die Identität der Produkte wird durch ESI-Massenspektrometrie bestätigt. Zyklisiertes Fengycin kann mittels präparativer HPLC rein dargestellt werden.

### Ausführungsbeispiel 2: Herstellung und Reinigung des Fengycin-Benzylmercaptan-Substrates sowie Zyklisierung

Herstellung, Reinigung und Zyklisierung des Fengycin-Benzylmercaptan-Substrates erfolgten analog zu Ausführungsbeispiel 1, wobei in Ausführungsbeispiel 2 0,05 mmol Benzylmercaptan an Stelle von 0,05 mMol Thiophenol eingesetzt werden. Die Ausbeute des zyklisierten Fengomycins beträgt etwa 70 %.

### Ausführungsbeispiel 3: Herstellung und Reinigung weiterer Fengycin- Substrate sowie Zyklisierung

Fengycin wird wie unter Ausführungsbeispiel 1 und 2 beschrieben mit weiteren erfindungsgemäßen Abgangsgruppen umgesetzt. Dabei handelt es sich um 2-Mercaptopyridin, p-Nitrothiophenol und Pentafluorthiophenol. Die Zyklisierung dieser Fengycin-Substrate erfolgt analog zu Ausführungsbeispiel 1 und ergibt deutlich höhere Anteile an nicht enzymatisch katalysiertem Zyklisierungsprodukt bzw. hydrolysiertem Produkt als im Falle der Verwendung von Thiophenol bzw. Benzylmercaptan.

**Tabelle 1.**

| Die linearen Peptide Fengycin, Surfactin, CDA und Syringomycin werden wie unter Ausführungsbeispiel 1 beschrieben mit Thiophenol umgesetzt und anschließend enzymatisch zyklisiert. Tab. 1 zeigt die Ergebnisse der massenspektrometrischen Messung der erhaltenen erfindungsgemäßen Substrate. | | | |
|---|---|---|---|
| Verbindung | Spezies | Ionisations-methode | Beobachtete Masse (berechnete Masse) (Da) |
| Fengycin-Thiophenol | [M+H]⁺ | ESI | 1361,40 (1361,60) |
| Surfactin-Thiophenol | [M+H]⁺ | ESI | 965,40 (965,49) |
| CDA-Thiophenol | [M+H]⁺ | ESI | 1519,30 (1519,5) |
| Syringomycin-Thiophenol | [M+H]⁺ | ESI | 1175,60 (1175,54) |

### [Abbildungslegenden]

### Fig. 1: HPLC der Reaktion von Fengycin-Thiophenol mit der Fengycin-Peptidzyklase

HPLC-MS mit einer reversed phase C₁₈ Nucleodur Säule (Macherey and Nagel, 250/3, Porendurchmesser:100 Å, Partikelgröße:3 µm) mit dem folgenden Gradienten: 0-35 min, 30-60 % Acetonitril/0.1 % TFA in WasserWasser/0.1 % TFA; 0.4 mL/min, 40°C. 1 zeigt die Kontrollreaktion mit einem mutierten (inaktivierten) Enzym. 2 zeigt die Inkubation mit dem nativen Enzym (aktiv). Su = Substrat, Cy = zyklisches Produkt, Hy = hydrolisiertes Produkt.

### Fig. 2: HPLC der Reaktion von Surfactin-Thiophenol mit der Surfactin-Peptidzyklase

HPLC-MS mit einer reversed phase C₁₈ Nucleodur Säule (Macherey and Nagel, 250/3, Porendurchmesser:100 Å, Partikelgröße:3 µm) mit dem folgenden Gradienten: 0-35 min, 30-60 % Acetonitril/0.1 % TFA in Wasser/0.1 % TFA; 0.4 mL/min, 40°C. 1 zeigt die Kontrollreaktion ohne Enzym. 2 zeigt die Inkubation mit dem nativen Enzym (aktiv). Su = Substrat, Cy = zyklisches Produkt, Hy = hydrolisiertes Produkt, (Cy) nicht enzymatisch katalysierte Zyklisierung über eine Amino Seitengruppe in der Peptidsequenz an Position 3 (Dap).

### Fig. 3: Fengycin-Peptidzyklase

5 µM der rekombinanten Fengycin-Peptidzyklase, die in vorhergehenden Untersuchungen keine Zyklisierungsaktivität mit konventionellen SNAC-Substraten zeigte, wird mit 100 µM Fengycin-Thiophenol für 10, 30, 40, 50, 60 min bei Raumtemperatur in 25 mM HEPES, 50 mM NaCl bei pH 7 in 50 µL Gesamtvolumen inkubiert. Mit dieser Messung wird der lineare Bereich für weitere kinetische Studien ermittelt. Die Reaktionen werden durch Zugabe von 35 µL TFA (4 % in Wasser) gestoppt und auf der analytischen HPLC mit einer C₁₈-Nucleodur Säule (Macherey and Nagel, 250/3, Porendurchmesser: 100 Å, Partikelgröße: 3 µm) mit dem folgenden Gradienten untersucht: 0-35 min, 30-60 % Acetonitril/0.1 % TFA in Wasser/0.1 % TFA; 0.4 mL/min, 40°C.
Kinetische Untersuchungen werden zu verschiedenen Zeitpunkten bei Substrat-Konzentrationen von 50 µM bis 1000 µM durchgeführt und die kinetischen Größen K_{M} und k_{cat} aus der Lineweaver-Burk-Auftragung entnommen. Für Fengycin-Thiophenol ergibt sich für die Zyklisierungsreaktion ein K_{M} von 461 uM und ein k_{cat} von 0,33 min⁻¹.

### Fig. 4: Surfactin-Peptidzyklase

Im Falle der Surfactin-Peptidzyklase existieren kinetische Referenzdaten mit einem SNAC-Substrat. Im Falle von Surfactin-Thiophenol wird für die Zyklisierungsreaktion ein K_{M} von 1 26 µM und ein k_{cat} von 5,6 min⁻¹ bestimmt, was einem k_{cat}/K_{M} Wert von 0,04 µM⁻¹ min⁻¹ entspricht. Verglichen damit ist die kinetische Effizienz von Surfactin-SNAC, repräsentiert durch den k_{cat}/K_{M} Wert 0,0029 µM⁻¹ min⁻¹, 14 mal niedriger als mit Surfactin-Thiophenol.

### Fig. 5: CDA-Peptidzyklase

Ein ähnliches Ergebnis erhält man für die Zyklisierung von CDA-Thiophenol mit der "Calcium Dependent Antibiotic" Peptidzyklase (CDA). Der K_{M} Wert für das Thiophenol-Substrat beträgt 10,7 µM, und der k_{cat} Wert beläuft sich auf 0,21 min⁻¹ Die kinetische Effizienz des Thiophenol-Substrates mit einem k_{cat}/K_{M} Wert von 0,02 µM⁻¹ min⁻¹ ist zehnfach größer verglichen mit dem k_{cat/}K_{M} Wert von dem SNAC Substrat (k_{cat}/K_{M}= 0,0021 µM⁻¹min⁻¹).

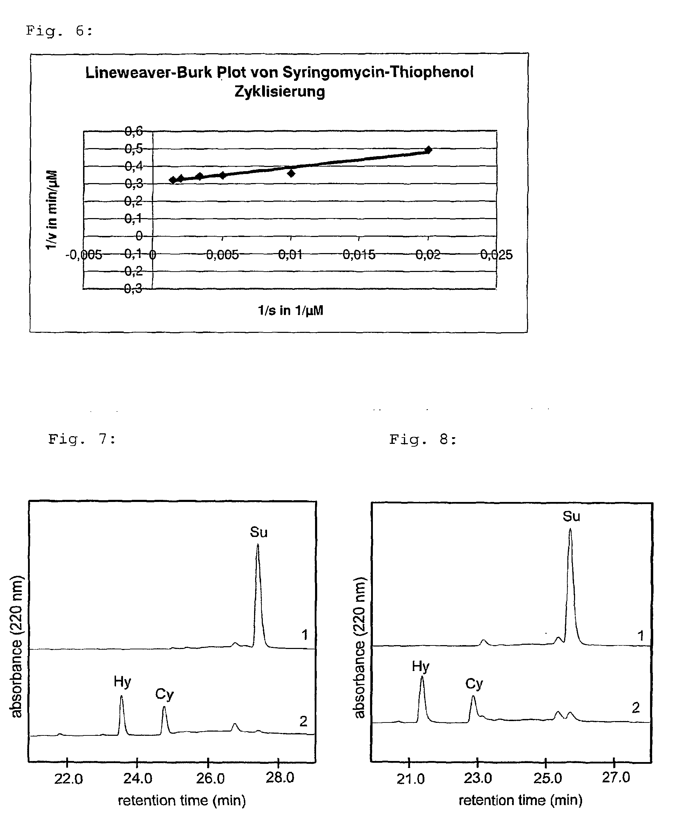

### Fig. 6: Syringomycin-Peptidzyklase

Im Falle der Syringomycin-Peptidzyklase existieren keine kinetischen Referenzdaten mit einem SNAC-Substrat, da dies in vorhergehenden Experimenten keine Aktivität zeigte. Im Falle von Syringomycin-Thiophenol wird für die Zyklisierungsreaktion ein K_{M} von 32,9 µM und ein k_{cat} von 0,805 min⁻¹ bestimmt, was einem k_{cat}/K_{M} Wert von 0,024 µM⁻¹ min⁻¹ entspricht.

### Fig. 7: HPLC der Reaktion von Surfactin-2-Thiokresol mit der Surfactin-Peptidzyklase

HPLC-MS mit einer reversed phase C₁₈ Nucleodur Säule (Macherey and Nagel, 250/3, Porendurchmesser:100 Å, Partikelgröße:3 µm) mit dem folgenden Gradienten: 0-35 min, 30-60 % Acetonitril/0.1 % TFA in Wasser /0.1 % TFA; 0.4 mL/min, 40°C.
1 zeigt die Kontrollreaktion mit einem mutierten (inaktivierten) Enzym. 2 zeigt die Inkubation mit dem nativen Enzym (aktiv). Su = Substrat, Cy = zyklisches Produkt, Hy = hydrolisiertes Produkt.

### Fig. 8: HPLC der Reaktion von Surfactin-4-Methoxyphenylthiol mit der Surfactin-Peptidzyklase

HPLC-MS mit einer reversed phase C₁₈ Nucleodur Säule (Macherey and Nagel, 250/3, Porendurchmesser:100 Å, Partikelgröße:3 µm) mit dem folgenden Gradienten: 0-35 min, 30-60 % Acetonitril/0.1 % TFA in Wasser/0.1 % TFA; 0.4 mL/min, 40°C.
1 zeigt die Kontrollreaktion mit einem mutierten (inaktivierten) Enzym. 2 zeigt die Inkubation mit dem nativen Enzym (aktiv). Su = Substrat, Cy = zyklisches Produkt, Hy = hydrolisiertes Produkt.

## Patentansprüche

1. Verfahren zur Herstellung zyklischer Peptide, bei dem
- eine Peptidzyklase mit einem linearen Peptid in Kontakt gebracht wird,
- das lineare Peptid einen Acylrest enthält, der durch eine chemisch mit diesem Acylrest verbundene nucleophile Abgangsgruppe aktiviert ist,
- der aktivierte Acylrest des linearen Peptids das Zentrum der Peptidzyklase selektiv acyliert, wobei die nucleophile Abgangsgruppe unter Bildung des zyklischen Peptids abgespalten wird und
- zyklische Peptide mit Ringen aus mindestens 5 Atomen gebildet werden,
**dadurch gekennzeichnet, dass**
- die nucleophile Abgangsgruppe, die mit dem Acylrest des linearen Peptids chemisch verbunden ist und diesen aktviert, ladungsstabilisiert ist und
- die ladungsstabilisierte Abgangsgruppe an die Acylgruppe der C-terminalen Carbonsäuregruppe des Peptids gebunden ist.

2. Verfahren zur Herstellung zyklischer Peptide gemäß Anspruch 1, **dadurch gekennzeichnet, dass** es sich bei den ladungsstabilisierten Abgangsgruppen um aromatische, heteroaromatische oder araliphatische Verbindungen handelt, bei denen eine Hydroxy- oder Thiogruppe an eines der Ringatome oder an ein an das Ringsystem gebundenes Kohlenstoffatom gebunden ist.

3. Verfahren zur Herstellung zyklischer Peptide nach einem der Ansprüche 1 oder 2, **dadurch gekennzeichnet, dass** es sich bei der Peptidzyklase um eine NRPS- oder PKS-Zyklase handelt, bevorzugt um eine gereinigte isolierte Thioesterase-Domäne.

4. Verfahren zur Herstellung zyklischer Peptide nach einem der Ansprüche 1 bis 3, **dadurch gekennzeichnet, dass** das lineare Peptid in seinem Rückgrat proteinogene und / oder nicht proteinogene Aminosäuren enthält, wobei in das Rückgrat auch Reste eingebettet sein können, die sich nicht von Aminosäuren ableiten.

5. Verfahren zur Herstellung zyklischer Peptide nach einem der Ansprüche 1 bis 4, **dadurch gekennzeichnet, dass** es sich bei der ladungsstabilisierten Abgangsgruppe um eine Verbindung der Formel 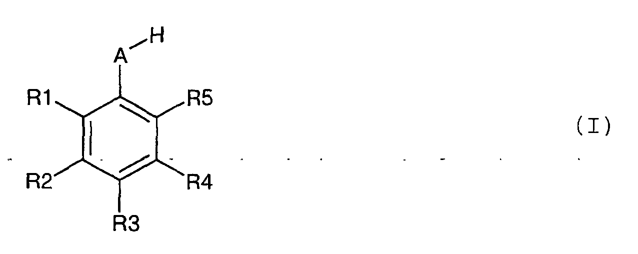 handelt, wobei gilt:
A = O, S
und wobei R1, R2, R3, R4 und R5 unabhängig voneinander sind:
-NO₂, -CN, -F, -Cl, -Br, -I, -CH₂Cl, -SO₃H, -H, -NH₃⁺, -NL₃⁺,-C(=O)L, -C(=O)Het, -O⁻ -NL₂, -NH₂, -OL, -OH, -NHC(=O)L,-OC(=O)L, -SL, -CO₂⁻, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind.

6. Verfahren zur Herstellung zyklischer Peptide nach einem der Ansprüche 1 bis 5, **dadurch gekennzeichnet, dass** es sich bei der ladungsstabilisierten Abgangsgruppe um eine Verbindung der Formel 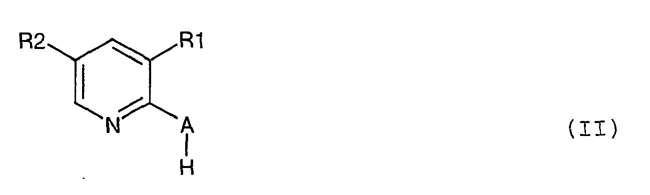 handelt, wobei gilt:
A = O, S
und wobei R1 und R2 unabhängig voneinander sind:
-NO₂, -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂⁻, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind.

7. Verfahren zur Herstellung zyklischer Peptide nach einem der Ansprüche 1 bis 6, **dadurch gekennzeichnet, dass** es sich bei der ladungsstabilisierten Abgangsgruppe um eine Verbindung der Formel 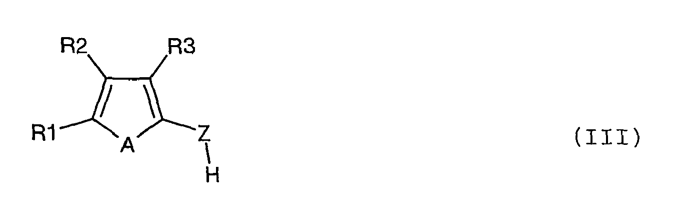 handelt, wobei gilt:
A = O , S und
Z = O, S,
und wobei R1, R2, und R3 unabhängig voneinander sind:
-NO₂, -CN, -F, -Cl, -Br, -I, -CH₂Cl, -SO₃H, -H, -NH₃⁺, -NL₃⁺,-C(=O)L, -C(=O)Het, -O⁻, -NL₂, -NH₂, -OL, -OH, -NHC(=O)L,-OC(=O)L, -SL, -CO₂-, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind.

8. Verfahren zur Herstellung zyklischer Peptide nach einem der Ansprüche 1 bis 7, **dadurch gekennzeichnet, dass** es sich bei der ladungsstabilisierten Abgangsgruppe um eine Verbindung der Formel 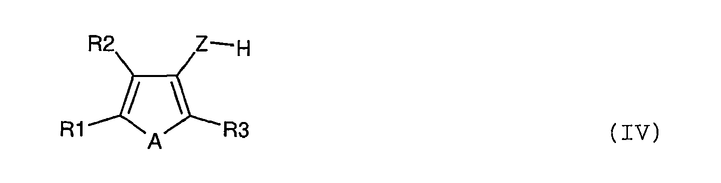 handelt, wobei gilt:
A = O , S und
Z = O, S,
und wobei R1, R2, und R3 unabhängig voneinander sind:
-NO₂, -CN, -F, -Cl, -Br, -I, -CH₂Cl, -SO₃H, -H, -NH₃⁺, -NL₃⁺ ,-C(=O)L, -C(=O)Het, -O⁻, -NL₂, -NH₂, -OL, -OH, -NHC(=O)L,-OC(=O)L, -SL, -CO₂⁻, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind.

9. Verfahren zur Herstellung zyklischer Peptide nach einem der Ansprüche 1 bis 8, **dadurch gekennzeichnet, dass** es sich bei der ladungsstabilisierten Abgangsgruppe um eine Verbindung der Formel 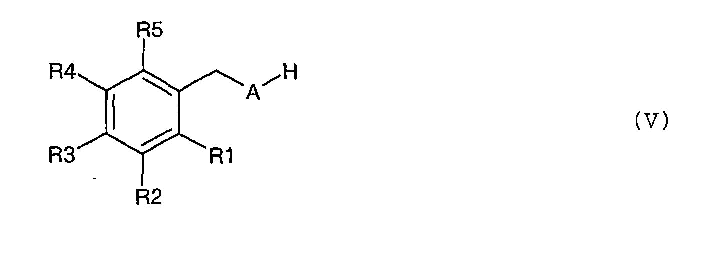 handelt, wobei gilt:
A = O, S
und wobei R1, R2, R3, R4 und R5 unabhängig voneinander sind: -NO₂, -CN, -F, -Cl, -Br, -I, -CH₂Cl, - SO₃ -H, -NH₃⁺, -NL₃⁺, -C(=O)L, -C(=O)Het, -O⁻, -NL₂, -NH₂, -OL, -OH, -NHC(=O)L,-OC(=O)L, -SL, -CO₂-, -Alkyl, -Alkenyl, -Cycloalkyl,-Cycloalkenyl, -Heteroalkyl, -Heterocycloalkyl, -Aryl, -Heteroaryl,
wobei
L = -Alkyl, -Alkenyl, -Cycloalkyl, -Cycloalkenyl, -Heteroalkyl, -Heterocycloalkyl, -Aryl, -Heteroaryl, wobei -Alkyl für eine Gruppe mit 1 bis 20 Kohlenstoffatomen und -Alkenyl für eine einfach oder mehrfach ungesättigte Gruppe mit 2 bis 20 Kohlenstoffatomen steht und -Alkyl bzw. -Alkenyl linear oder verzweigt sind, -Cycloalkyl und -Cycloalkenyl für eine Gruppe mit 3 bis 20 Kohlenstoffatomen steht, Heteroalkyl für eine Alkylgruppe steht, worin bis zu 5 Kohlenstoffatome ausgewählt aus der Gruppe Stickstoff, Sauerstoff, Schwefel, Phosphor ersetzt sind, die heterocyclischen Gruppen für einen Rest mit 1 bis 20 Kohlenstoffatomen stehen, worin bis zu 5 Kohlenstoffatome durch Heteroatome ausgewählt aus der Gruppe Stickstoff, Sauerstoff, Schwefel, Phosphor ersetzt sind, Aryl für einen aromatischen Rest mit 5 bis 20 Kohlenstoffatomen und Heteroaryl für einen entsprechenden aromatischen Rest steht, in dem bis zu 5 Kohlenstoffatome durch Heteroatome, ausgewählt aus der Gruppe Stickstoff, Sauerstoff, Schwefel, Phosphor, ersetzt sind.

10. Verfahren zur Herstellung eines Substrates und anschließende Umsetzung dieses Substrates mit Peptid-Zyklasen zu einem zyklischen Peptid, wobei die Substrate lineare Peptide sind, **dadurch gekennzeichnet, dass** nacheinander folgende Schritte ausgeführt werden:
- Versetzen der freien Peptidsäure mit einem den C-Terminus der Peptidsäure aktivierenden Reagenz, einem Kupplungsadditiv und einer ladungsstabilisierten Abgangsgruppe in einem Lösungsmittel
- Rühren bei Raumtemperatur,
- Zugabe einer Base und weiteres Rühren bei Raumtemperatur,
- Filtrieren,
- Entfernen des Lösungsmittels,
- Entschützen des Peptids,
- Zugabe einer Peptid-Zyklase,
- Reinigung des erhaltenen zyklischen Peptids.

11. Verfahren zur Herstellung eines Substrates und anschließende Umsetzung dieses Substrates mit Peptid-Zyklasen zu einem zyklischen Peptid gemäß Anspruch 10, **dadurch gekennzeichnet, dass** die Acylgruppe der C-terminalen Aminosäure des linearen Peptids an eine Abgangsgruppe nach einem der Ansprüche 5 bis 9 gebunden ist.

12. Verfahren zur Herstellung eines Substrates und anschließende Umsetzung dieses Substrates mit Peptid-Zyklasen zu einem zyklischen Peptid gemäß Anspruch 11, **dadurch gekennzeichnet, dass** die Abgangsgruppe einen pK_{A}-Wert kleiner oder gleich 10, bevorzugt kleiner oder gleich 8 besitzt.

13. Verfahren zur Herstellung eines Substrates und anschließende Umsetzung dieses Substrates mit Peptid-Zyklasen zu einem zyklischen Peptid gemäß nach einem der Ansprüche 10 bis 12, **dadurch gekennzeichnet, dass** als Aktivierungsreagenz für den freien C-Terminus oder eine Seitenketten-Carbonsäure der Peptidcarbonsäure DCC, DCI, PyClop, HBTU, HATU, HOSu, TBTU, T3P, BopCl oder 3-Cl-1-Pyridiniumiodid verwendet wird.

14. Verfahren zur Herstellung eines Substrates und anschließende Umsetzung dieses Substrates mit Peptid-Zyklasen zu einem zyklischen Peptid gemäß einem der Ansprüche 10 bis 13, **dadurch gekennzeichnet, dass** als Kupplungsadditiv HOBt, HOAt oder HONB verwendet wird.

15. Verwendung von ladungsstabilisierten Abgangsgruppen gemäß einem der Ansprüche 1 bis 14 in einem Kit zur Herstellung zyklischer Peptide.

## Claims

1. Method for the production of cyclic peptides, in which
- a peptide cyclase is brought in contact with a linear peptide,
- the linear peptide contains an acyl residue, which is activated by a nucleophilic leaving group bound chemically with this acyl residue,
- the activated acyl residue of the linear peptide selectively acylates the center of the peptide cyclase, wherein the nucleophilic leaving group is cleaved off during formation of the cyclic peptide and
- cyclic peptides with rings of at least 5 atoms are formed,
**characterized in that**
- the nucleophilic leaving group, which is chemically bound to the acyl residue of the linear peptide and which activates the latter, is charge-stabilized and
- the charge-stabilized leaving group is bound to the acyl group of the C-terminal carboxylic acid group of the peptide.

2. Method for the production of cyclic peptides according to claim 1, **characterized in that** the charge-stabilized leaving groups are aromatic, heteroaromatic or araliphatic compounds, on which a hydroxy or thio group is bound to one of the ring atoms or to a carbon atom bound to the ring system.

3. Method for the production of cyclic peptides according to either of claims 1 and 2, **characterized in that** the peptide cyclase is a NRPS or PKS cyclase, preferably a purified, isolated thioesterase domain.

4. Method for the production of cyclic peptides according to any of claims 1 to 3, **characterized in that** the linear peptide contains proteinogenic and/or non-proteinogenic amino acids in its backbone, wherein residues which do not derive from amino acids can also be embedded in the backbone.

5. Method for the production of cyclic peptides according to any of claims 1 to 4, **characterized in that** the charge-stabilized leaving group is a compound of the formula 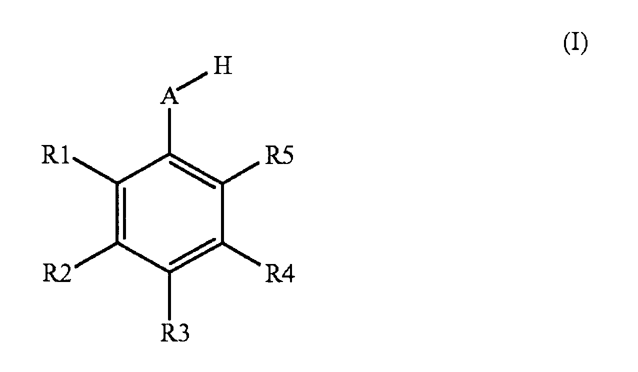 wherein applies:
A = O, S
and wherein R1, R2, R3, R4 and R5 are independently of one another:
-NO₂, -CN, -F, -Cl, -Br, -I, -CH₂Cl, -SO₃H, -H, -NH₃⁺, -NL₃⁺, -C(=O)L, -C(=O)Het, -O⁻, -NL₂, -NH₂, -OL, -OH, -NHC(=O)L, -OC(=O)L, -SL, -CO₂⁻, -alkyl, -alkenyl, -cycloalkyl, -cycloalkenyl, -heteroalkyl, -heterocycloalkyl, -aryl, -heteroaryl,
wherein
L = -alkyl, -alkenyl, -cycloalkyl, -cycloalkenyl, -heteroalkyl, -heterocycloalkyl, -aryl, -heteroaryl, wherein -alkyl stands for a group with 1 to 20 carbon atoms and -alkenyl for a monounsaturated or polyunsaturated group with 2 to 20 carbon atoms and -alkyl or -alkenyl are linear or branched; -cycloalkyl and -cycloalkenyl stand for a group with 3 to 20 carbon atoms; heteroalkyl stands for an alkyl group wherein up to 5 carbon atoms are replaced by heteroatoms chosen from the group nitrogen, oxygen, sulfur, phosphorus; the heterocyclic groups stand for a residue with 1 to 20 carbon atoms wherein up to 5 carbon atoms are replaced by heteroatoms chosen from the group nitrogen, oxygen, sulfur, phosphorus; aryl stands for an aromatic residue with 5 to 20 carbon atoms and heteroaryl stands for a corresponding aromatic residue in which up to 5 carbon atoms are replaced by heteroatoms chosen from the group nitrogen, oxygen, sulfur, phosphorus.

6. Method for the production of cyclic peptides according to any of claims 1 to 5, **characterized in that** the charge-stabilized leaving group is a compound of the formula 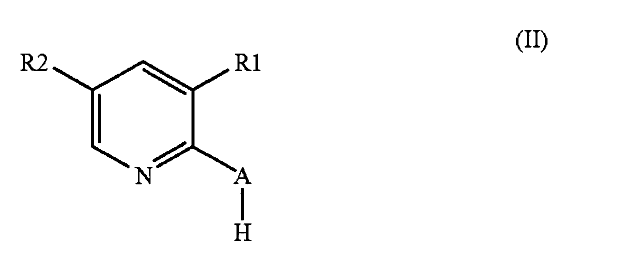 wherein applies:
A = O, S
and wherein R1 and R2 are independently of one another:
-NO₂, -CN, -F, -Cl, -Br, -I, -CH₂Cl, -SO₃H, -H, -NH₃⁺, -NL₃-, -C(=O)L, -C(=O)Het, -O⁻, -NL₂, -NH₂, -OL, -OH, -NHC(=O)L, -OC(=O)L, -SL, -CO₂⁻, -alkyl, -alkenyl, -cycloalkyl, -cycloalkenyl, -heteroalkyl, -heterocycloalkyl, -aryl, -heteroaryl,
wherein
L = -alkyl, -alkenyl, -cycloalkyl, -cycloalkenyl, -heteroalkyl, -heterocycloalkyl, -aryl, -heteroaryl, wherein -alkyl stands for a group with 1 to 20 carbon atoms and -alkenyl for a monounsaturated or polyunsaturated group with 2 to 20 carbon atoms and -alkyl or -alkenyl are linear or branched; -cycloalkyl and -cycloalkenyl stand for a group with 3 to 20 carbon atoms; heteroalkyl stands for an alkyl group wherein up to 5 carbon atoms are replaced by heteroatoms chosen from the group nitrogen, oxygen, sulfur, phosphorus; the heterocyclic groups stand for a residue with 1 to 20 carbon atoms wherein up to 5 carbon atoms are replaced by heteroatoms chosen from the group nitrogen, oxygen, sulfur, phosphorus; aryl stands for an aromatic residue with 5 to 20 carbon atoms and heteroaryl stands for a corresponding aromatic residue in which up to 5 carbon atoms are replaced by heteroatoms chosen from the group nitrogen, oxygen, sulfur, phosphorus.

7. Method for the production of cyclic peptides according to any of claims 1 to 6, **characterized in that** the charge-stabilized leaving group is a compound of the formula 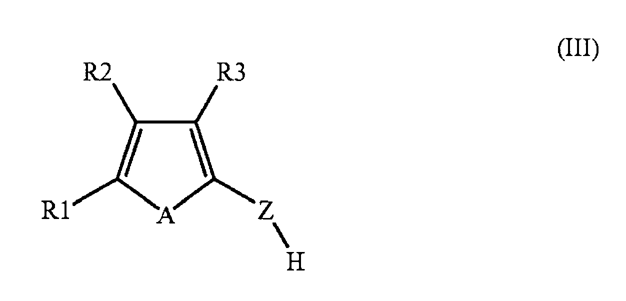 wherein applies:
A = O , S and
Z = O, S,
and wherein R1, R2, and R3 are independently of one another:
-NO₂, -CN, -F, -Cl, -Br, -I, -CH₂Cl, -SO₃H, -H, -NH₃⁺, -NL₃⁺, -C(=O)L, -C(=O)Het, -O⁻, -NL₂, -NH₂, -OL, -OH, -NHC(=O)L, -OC(=O)L, -SL, -CO₂⁻, -alkyl, -alkenyl, -cycloalkyl, -cycloalkenyl, -heteroalkyl, -heterocycloalkyl, -aryl, -heteroaryl, wherein L = -alkyl, -alkenyl, -cycloalkyl, -cycloalkenyl, -heteroalkyl, -heterocycloalkyl, -aryl, -heteroaryl, wherein -alkyl stands for a group with 1 to 20 carbon atoms and -alkenyl for a monounsaturated or polyunsaturated group with 2 to 20 carbon atoms and -alkyl or -alkenyl are linear or branched; -cycloalkyl and -cycloalkenyl stand for a group with 3 to 20 carbon atoms; heteroalkyl stands for an alkyl group wherein up to 5 carbon atoms are replaced by heteroatoms chosen from the group nitrogen, oxygen, sulfur, phosphorus; the heterocyclic groups stand for a residue with 1 to 20 carbon atoms wherein up to 5 carbon atoms are replaced by heteroatoms chosen from the group nitrogen, oxygen, sulfur, phosphorus; aryl stands for an aromatic residue with 5 to 20 carbon atoms and heteroaryl stands for a corresponding aromatic residue in which up to 5 carbon atoms are replaced by heteroatoms chosen from the group nitrogen, oxygen, sulfur, phosphorus.

8. Method for the production of cyclic peptides according to any of claims 1 to 7, **characterized in that** the charge-stabilized leaving group is a compound of the formula 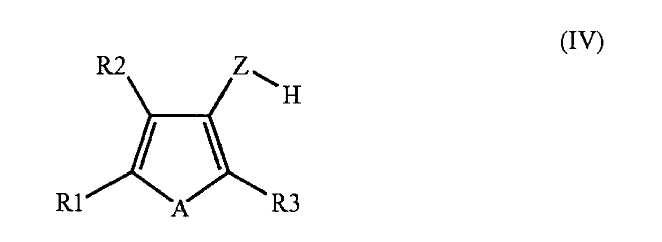 wherein applies:
A = O , S and
Z = O, S,
and wherein R1, R2, and R3 are independently of one another:
-NO₂, -CN, -F, -Cl, -Br, -I, -CH₂Cl, -SO₃H, -H, -NH₃⁺, -NL₃⁺, -C(=O)L, -C(=O)Het, -O⁻, -NL₂, -NH₂, -OL, -OH, -NHC(=O)L, -OC(=O)L, -SL, -CO₂⁻, -alkyl, -alkenyl, -cycloalkyl, -cycloalkenyl, -heteroalkyl, -heterocycloalkyl, -aryl, -heteroaryl,
wherein
L = -alkyl, -alkenyl, -cycloalkyl, -cycloalkenyl, -heteroalkyl, -heterocycloalkyl, -aryl, -heteroaryl, wherein -alkyl stands for a group with 1 to 20 carbon atoms and -alkenyl for a monounsaturated or polyunsaturated group with 2 to 20 carbon atoms and -alkyl or -alkenyl are linear or branched; -cycloalkyl and -cycloalkenyl stand for a group with 3 to 20 carbon atoms; heteroalkyl stands for an alkyl group wherein up to 5 carbon atoms are replaced by heteroatoms chosen from the group nitrogen, oxygen, sulfur, phosphorus; the heterocyclic groups stand for a residue with 1 to 20 carbon atoms wherein up to 5 carbon atoms are replaced by heteroatoms chosen from the group nitrogen, oxygen, sulfur, phosphorus; aryl stands for an aromatic residue with 5 to 20 carbon atoms and heteroaryl stands for a corresponding aromatic residue in which up to 5 carbon atoms are replaced by heteroatoms chosen from the group nitrogen, oxygen, sulfur, phosphorus.

9. Method for the production of cyclic peptides according to any of claims 1 to 8, **characterized in that** the charge-stabilized leaving group is a compound of the formula 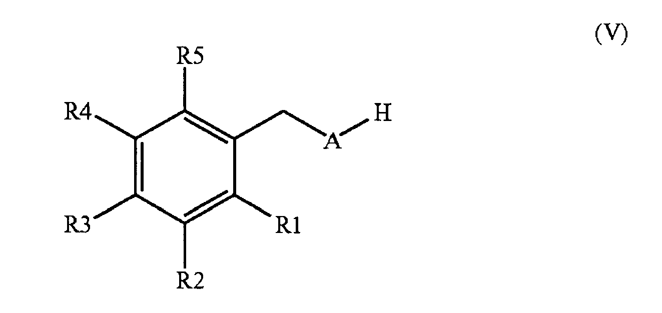 wherein applies:
A = O, S
and wherein R1 R2, R3, R4 and R5 are independently of one another:
-NO₂, -CN, -F, -Cl, -Br, -I, -CH₂Cl, -SO₃H, -H, -NH₃⁺, -NL₃⁺, -C(=O)L, -C(=O) Het, -O⁻, -NL₂, -NH₂, -OL, -OH, -NHC(=O)L, -OC(=O)L, -SL, -CO₂⁻, -alkyl, -alkenyl, -cycloalkyl, -cycloalkenyl, -heteroalkyl, -heterocycloalkyl, -aryl, -heteroaryl, wherein L=-alkyl, -alkenyl, -cycloalkyl, -cycloalkenyl, -heteroalkyl, -heterocycloalkyl, -aryl, -heteroaryl, wherein -alkyl stands for a group with 1 to 20 carbon atoms and -alkenyl for a monounsaturated or polyunsaturated group with 2 to 20 carbon atoms and -alkyl or -alkenyl are linear or branched; -cycloalkyl and -cycloalkenyl stand for a group with 3 to 20 carbon atoms; heteroalkyl stands for an alkyl group wherein up to 5 carbon atoms are replaced by heteroatoms chosen from the group nitrogen, oxygen, sulfur, phosphorus; the heterocyclic groups stand for a residue with 1 to 20 carbon atoms wherein up to 5 carbon atoms are replaced by heteroatoms chosen from the group nitrogen, oxygen, sulfur, phosphorus; aryl stands for an aromatic residue with 5 to 20 carbon atoms and heteroaryl stands for a corresponding aromatic residue in which up to 5 carbon atoms are replaced by heteroatoms chosen from the group nitrogen, oxygen, sulfur, phosphorus.

10. Method for the production of a substrate and subsequent reaction of this substrate with peptide cyclases into a cyclic peptide, wherein the substrates are linear peptides, **characterized in that** the following steps are carried out one after the other:
- Adding a reagent activating the C-terminus of the peptide acid, a coupling additive and a charge-stabilized leaving group to the free peptide acid in a solvent
- Stirring at room temperature,
- Addition of a base and further stirring at room temperature,
- Filtration,
- Removal of the solvent,
- Deprotection of the peptide,
- Addition of a peptide cyclase,
- Purification of the cyclic peptide obtained.

11. Method for the production of a substrate and subsequent reaction of this substrate with peptide cyclases into a cyclic peptide according to claim 10, **characterized in that** the acyl group of the C-terminal amino acid of the linear peptide is bound to a leaving group according to any of claims 5 to 9.

12. Method for the production of a substrate and subsequent reaction of this substrate with peptide cyclases into a cyclic peptide according to claim 11, **characterized in that** the leaving group possesses a pK_{A} value less than or equal to 10, preferably less than or equal to 8.

13. Method for the production of a substrate and subsequent reaction of this substrate with peptide cyclases into a cyclic peptide according to any of claims 10 to 12, **characterized in that** DCC, DCI, PyClop, HBTU, HATU, HOSu, TBTU, T3P, BopCl or 3-Cl-1-pyridinium iodide is used as an activation reagent for the free C-terminus or a side chain carboxylic acid of the peptide carboxylic acid.

14. Method for the production of a substrate and subsequent reaction of this substrate with peptide cyclases into a cyclic peptide according to any of claims 10 to 13, **characterized in that** HOBt, HOAt or HONB is used as a coupling additive.

15. Use of charge-stabilized leaving groups according to any of claims 1 to 14 in a kit for the production of cyclic peptides.

## Revendications

1. Procédé de fabrication d'un peptide cyclique, selon lequel
- une peptide cyclase est mise en contact avec un peptide linéaire,
- le peptide linéaire contient un radical acyle, qui est activé par un groupement partant nucléophile relié chimiquement à ce radical acyle,
- le radical acyle activé du peptide linéaire acyle sélectivement le centre de la peptide cyclase, le groupement partant étant scindé lors de la formation du peptide cyclique et
- le peptide cyclique est formé avec des cycles d'au moins 5 atomes,
**caractérisé en ce que**
- le groupement partant nucléophile, qui est relié chimiquement au radical acyle du peptide linéaire et qui l'active, est stabilisé par des charges et **en ce que**
- le groupement partant stabilisé par des charges est relié au groupement acyle du groupement acide carboxylique de la terminaison C du peptide.

2. Procédé de fabrication d'un peptide cyclique selon la revendication 1, **caractérisé en ce que** le groupement partant stabilisé par des charges est un composé aromatique, hétéroaromatique ou araliphatique comprenant un groupement hydroxy ou thio relié à un atome cyclique ou à un atome de carbone relié au système cyclique.

3. Procédé de fabrication d'un peptide cyclique selon l'une quelconque des revendications 1 ou 2, **caractérisé en ce que** la peptide cyclase est une cyclase NRPS ou PKS, de préférence un domaine thioestérase isolé et purifié.

4. Procédé de fabrication d'un peptide cyclique selon l'une quelconque des revendications 1 à 3, **caractérisé en ce que** le peptide linéaire contient dans son squelette des acides aminés protéinogènes et/ou non protéinogènes, des radicaux ne dérivant pas d'acides aminés pouvant également être incorporés dans le squelette.

5. Procédé de fabrication d'un peptide cyclique selon l'une quelconque des revendications 1 à 4, **caractérisé en ce que** le groupement partant stabilisé par des charges est un composé de formule 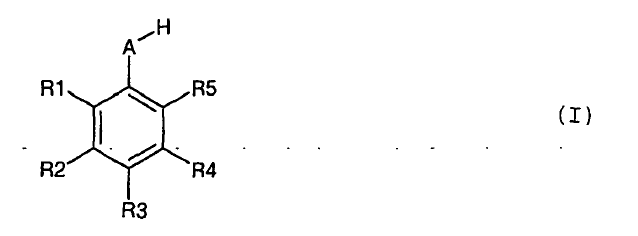 dans laquelle : A = O, S
et dans laquelle R1, R2, R3, R4 et R5 représentent indépendamment les uns des autres : -NO₂, -CN, -F, -Cl, -Br, -1, -CH₂Cl, -SO₃H, -H, -NH₃⁺, -NL₃⁺, -C(=O)L,-C(=O)Het, -O⁻ , -NL₂, -NH₂, -OL, -OH, -NHC(=O)L,-OC(=O)L, -SL, -CO₂⁻, -alkyle, -alcényle, -cycloalkyle,-cycloalcényle, -hétéroalkyle, -hétérocycloalkyle,-aryle, -hétéroaryle, avec
L = -alkyle, -alcényle, -cycloalkyle, -cycloalcényle,-hétéroalkyle, -hétérocycloalkyle, -aryle, -hétéroaryle, avec -alkyle représentant un groupement ayant de 1 à 20 atomes de carbone et -alcényle représentant un groupement à une ou plusieurs insaturations ayant de 2 à 20 atomes de carbone et -alkyle et alcényle étant linéaires ou ramifiés, -cycloalkyle et -cycloalcényle représentant un groupement ayant de 3 à 20 atomes de carbone, hétéroalkyle représentant un groupement alkyle dans lequel jusqu'à 5 atomes de carbone sont remplacés par des hétéroatomes choisis dans le groupement azote, oxygène, soufre, phosphore, les groupements hétérocycliques représentent un radical ayant de 1 à 20 atomes de carbone dans lequel jusqu'à 5 atomes de carbone sont remplacés par des hétéroatomes choisis dans le groupe azote, oxygène, soufre, phosphore, aryle représente un radical aromatique ayant de 5 à 20 atomes de carbone et hétéroaryle représente un radical aromatique correspondant dans lequel jusqu'à 5 atomes de carbone sont remplacés par des hétéroatomes choisis dans le groupe azote, oxygène, soufre, phosphore.

6. Procédé de fabrication d'un peptide cyclique selon l'une quelconque des revendications 1 à 5, **caractérisé en ce que** le groupement partant stabilisé par des charges est un composé de formule 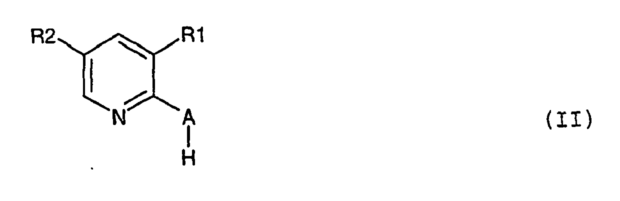 dans laquelle: A = O, S
et dans laquelle R1 et R2 représentent indépendamment l'un de l'autre -NO₂, -CN, -F, -Cl, -Br, -I, -CH₂Cl,-SO₃H, -H, -NH₃⁺, -NL₃⁺, -C(=O)L, -C(=O)Het, -O⁻, -NL₂,-NH₂, -OL, -OH, -NHC(=O)L, -OC(=O)L, -SL, -CO₂⁻, -alkyle, -alcényle, -cycloalkyle, -cycloalcényle, -hétéroalkyle, -hétérocycloalkyle, -aryle, -hétéroaryle, avec
L = -alkyle, -alcényle, -cycloalkyle, -cycloalcényle, - hétéroalkyle, -hétérocycloalkyle, -aryle, -hétéroaryle, avec -alkyle représentant un groupement ayant de 1 à 20 atomes de carbone et -alcényle représentant un groupement à une ou plusieurs insaturations ayant de 2 à 20 atomes de carbone et -alkyle et alcényle étant linéaires ou ramifiés, -cycloalkyle et -cycloalcényle représentant un groupement ayant de 3 à 20 atomes de carbone, hétéroalkyle représentant un groupement alkyle dans lequel jusqu'à 5 atomes de carbone sont remplacés par des hétéroatomes choisis dans le groupement azote, oxygène, soufre, phosphore, les groupements hétérocycliques représentent un radical ayant de 1 à 20 atomes de carbone dans lequel jusqu'à 5 atomes de carbone sont remplacés par des hétéroatomes choisis dans le groupe azote, oxygène, soufre, phosphore, aryle représente un radical aromatique ayant de 5 à 20 atomes de carbone et hétéroaryle représente un radical aromatique correspondant dans lequel jusqu'à 5 atomes de carbone sont remplacés par des hétéroatomes choisis dans le groupe azote, oxygène, soufre, phosphore.

7. Procédé de fabrication d'un peptide cyclique selon l'une quelconque des revendications 1 à 6, **caractérisé en ce que** le groupement partant stabilisé par des charges est un composé de formule 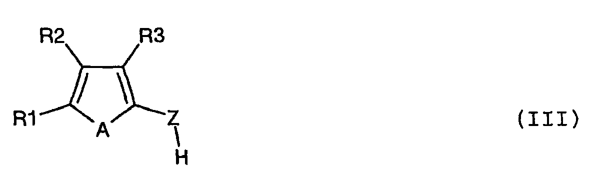 dans laquelle :
A = O, S et
Z = O, S,
et dans laquelle R1, R2 et R3 représentent indépendamment l'un de l'autre :
-NO₂, -CN, -F, -Cl, -Br, -I, -CH₂Cl, -SO₃H, -H, -NH₃⁺,-NL₃⁺, -C(=O)L, -C(=O)Het, -O⁻, -NL₂, -NH₂, -OL, -OH,-NHC(=O)L, -OC(=O)L, -SL, -CO₂⁻, -alkyle, -alcényle,-cycloalkyle, -cycloalcényle, -hétéroalkyle,-hétérocycloalkyle, -aryle, -hétéroaryle,
avec
L = -alkyle, -alcényle, -cycloalkyle, -cycloalcényle,-hétéroalkyle, -hétérocycloalkyle, -aryle, -hétéroaryle, avec -alkyle représentant un groupement ayant de 1 à 20 atomes de carbone et -alcényle représentant un groupement à une ou plusieurs insaturations ayant de 2 à 20 atomes de carbone et -alkyle et alcényle étant linéaires ou ramifiés, -cycloalkyle et -cycloalcényle représentant un groupement ayant de 3 à 20 atomes de carbone, hétéroalkyle représentant un groupement alkyle dans lequel jusqu'à 5 atomes de carbone sont remplacés par des hétéroatomes choisis dans le groupement azote, oxygène, soufre, phosphore, les groupements hétérocycliques représentent un radical ayant de 1 à 20 atomes de carbone dans lequel jusqu'à 5 atomes de carbone sont remplacés par des hétéroatomes choisis dans le groupe azote, oxygène, soufre, phosphore, aryle représente un radical aromatique ayant de 5 à 20 atomes de carbone et hétéroaryle représente un radical aromatique correspondant dans lequel jusqu'à 5 atomes de carbone sont remplacés par des hétéroatomes choisis dans le groupe azote, oxygène, soufre, phosphore.

8. Procédé de fabrication d'un peptide cyclique selon l'une quelconque des revendications 1 à 7, **caractérisé en ce que** le groupement partant stabilisé par des charges est un composé de formule 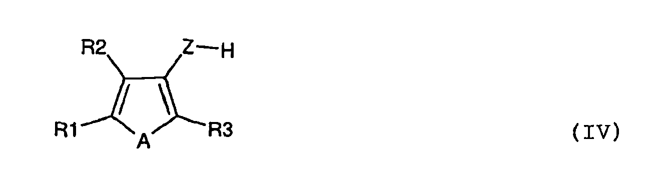 dans laquelle :
A = O, S et
Z = O, S,
et dans laquelle R1, R2 et R3 représentent indépendamment l'un de l'autre:
-NO₂, -CN, -F, -Cl, -Br, -I, -CH₂Cl, -SO₃H, -H, -NH₃⁺,-NL₃⁺, -C(=O)L, -C(=O)Het, -O , -NL₂, -NH₂, -OL, -OH,-NHC(=O)L, -OC(=O)L, -SL, -CO₂⁻, -alkyle, -alcényle,-cycloalkyle, -cycloalcényle, -hétéroalkyle,-hétérocycloalkyle, -aryle, -hétéroaryle, avec
L = -alkyle, -alcényle, -cycloalkyle, -cycloalcényle,-hétéroalkyle, -hétérocycloalkyle, -aryle, -hétéroaryle, avec -alkyle représentant un groupement ayant de 1 à 20 atomes de carbone et -alcényle représentant un groupement à une ou plusieurs insaturations ayant de 2 à 20 atomes de carbone et -alkyle et alcényle étant linéaires ou ramifiés, -cycloalkyle et -cycloalcényle représentant un groupement ayant de 3 à 20 atomes de carbone, hétéroalkyle représentant un groupement alkyle dans lequel jusqu'à 5 atomes de carbone sont remplacés par des hétéroatomes choisis dans le groupement azote, oxygène, soufre, phosphore, les groupements hétérocycliques représentent un radical ayant de 1 à 20 atomes de carbone dans lequel jusqu'à 5 atomes de carbone sont remplacés par des hétéroatomes choisis dans le groupe azote, oxygène, soufre, phosphore, aryle représente un radical aromatique ayant de 5 à 20 atomes de carbone et hétéroaryle représente un radical aromatique correspondant dans lequel jusqu'à 5 atomes de carbone sont remplacés par des hétéroatomes choisis dans le groupe azote, oxygène, soufre, phosphore.

9. Procédé de fabrication d'un peptide cyclique selon l'une quelconque des revendications 1 à 8, **caractérisé en ce que** le groupement partant stabilisé par des charges est un composé de formule 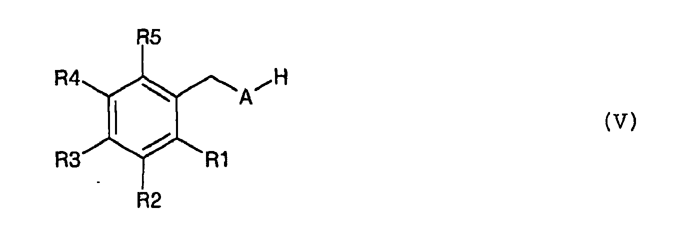 dans laquelle : A = O, S
et dans laquelle R1, R2, R3, R4 et R5 représentent indépendamment les uns des autres : -NO₂, -CN, -F, -Cl, -Br, -I, -CH₂Cl, -SO₃H, -H, -NH₃⁺, -NL₃⁺, -C(=O)L,-C(=O)Het, -O⁻, -NL₂, -NH₂, -OL, -OH, -NHC(=O)L,-OC(=O)L, -SL, -CO₂⁻, -alkyle, -alcényle, -cycloalkyle,-cycloalcényle, -hétéroalkyle, -hétérocycloalkyle,-aryle, -hétéroaryle, avec
L = -alkyle, -alcényle, -cycloalkyle, -cycloalcényle,-hétéroalkyle, -hétérocycloalkyle, -aryle, -hétéroaryle, avec -alkyle représentant un groupement ayant de 1 à 20 atomes de carbone et -alcényle représentant un groupement à une ou plusieurs insaturations ayant de 2 à 20 atomes de carbone et -alkyle et alcényle étant linéaires ou ramifiés, -cycloalkyle et -cycloalcényle représentant un groupement ayant de 3 à 20 atomes de carbone, hétéroalkyle représentant un groupement alkyle dans lequel jusqu'à 5 atomes de carbone sont remplacés par des hétéroatomes choisis dans le groupement azote, oxygène, soufre, phosphore, les groupements hétérocycliques représentent un radical ayant de 1 à 20 atomes de carbone dans lequel jusqu'à 5 atomes de carbone sont remplacés par des hétéroatomes choisis dans le groupe azote, oxygène, soufre, phosphore, aryle représente un radical aromatique ayant de 5 à 20 atomes de carbone et hétéroaryle représente un radical aromatique correspondant dans lequel jusqu'à 5 atomes de carbone sont remplacés par des hétéroatomes choisis dans le groupe azote, oxygène, soufre, phosphore.

10. Procédé de fabrication d'un substrat et de réaction ultérieure de ce substrat avec une peptide cyclase pour former un peptide cyclique, le substrat étant un peptide linéaire, **caractérisé en ce que** les étapes suivantes sont réalisées successivement :
- mélange de l'acide peptidique libre avec un réactif activant la terminaison C de l'acide peptidique, un additif de couplage et un groupement partant stabilisé par des charges dans un solvant
- agitation à température ambiante,
- ajout d'une base et agitation supplémentaire à température ambiante,
- filtration,
- élimination du solvant,
- déprotection du peptide,
- ajout d'une peptide cyclase,
- purification du peptide cyclique obtenu.

11. Procédé de fabrication d'un substrat et réaction ultérieure de ce substrat avec une peptide cyclase pour former un peptide cyclique selon la revendication 10, **caractérisé en ce que** le groupement acyle de l'acide aminé de la terminaison C du peptide linéaire est relié à un groupement partant selon l'une quelconque des revendications 5 à 9.

12. Procédé de fabrication d'un substrat et réaction ultérieure de ce substrat avec une peptide cyclase pour former un peptide cyclique selon la revendication 11, **caractérisé en ce que** le groupement partant a une valeur de pK_{A} inférieure ou égale à 10, de préférence inférieure ou égale à 8.

13. Procédé de fabrication d'un substrat et réaction ultérieure de ce substrat avec une peptide cyclase pour former un peptide cyclique selon l'une quelconque des revendications 10 à 12, **caractérisé en ce qu'**en tant que réactif d'activation pour la terminaison C libre ou un acide carboxylique sur une chaîne latérale de l'acide peptidique, DCC, DCI, PyClop, HBTU, HATU, HOSu, TBTU, T3P, BopCl ou iodure de 3-C1-1-pyridinium est utilisé.

14. Procédé de fabrication d'un substrat et réaction ultérieure de ce substrat avec une peptide cyclase pour former un peptide cyclique selon l'une quelconque des revendications 10 à 13, **caractérisé en ce qu'**en tant qu'additif de couplage, HOBt, HOAt ou HONB est utilisé.

15. Utilisation de groupements partants stabilisés par des charges selon l'une quelconque des revendications 1 à 14, dans un kit pour la fabrication d'un peptide cyclique.
